# EUROPEAN PATENT SPECIFICATION

(11) **EP 3 361 936 B1**
(45) Date of publication and mention of the grant of the patent: **18.02.2026**
(21) Application number: 16856307.0
(22) Date of filing: 14.10.2016
(51) Int. Cl.: A61N 7/00, A61B 5/00, A61B 17/00, A61B 17/225, A61N 7/02, A61B 18/00

(54) **ULTRASOUND DEVICE FOR VULVOVAGINAL REJUVENATION**
ULTRASCHALLVORRICHTUNG ZUR VULVOVAGINALEN VERJÜNGUNG
DISPOSITIF À ULTRASONS POUR RAJEUNISSEMENT VULVOVAGINAL

(30) Priority: 16.10.2015 US 201562242370 P; 30.06.2016 US 201662357098 P; 22.08.2016 US 201662378044 P
(43) Date of publication of application: 22.08.2018
(73) Proprietor: Madorra Inc., Portland, OR 97205 (US)
(72) Inventor: KRONE, Ryan Taylor, Portland, OR 97205 (US); ROCKWEILER, Holly Elizabeth, Portland, OR 97205 (US); JONES, Lauren Paige, Portland, OR 97205 (US); STEINBERGER, Jonathan Daniel, Portland, OR 97205 (US)
(74) Representative: J A Kemp LLP
(86) International application number: PCT/US2016/057119
(87) International publication number: WO 2017/066625

(56) References cited:
- WO-A1-2013/184798
- WO-A1-2015/116512
- WO-A1-2015/116512
- WO-A2-2007/092610
- US-A1- 2005 203 399
- US-A1- 2006 235 303
- US-A1- 2011 313 293
- US-A1- 2012 143 062
- US-A1- 2013 338 545
- US-A1- 2014 257 145
- US-A1- 2015 135 840

## Description

### FIELD

This invention relates generally to handheld ultrasound devices for use in the genital area for treating vulvovaginal atrophy.

### BACKGROUND

Vulvovaginal atrophy is an inflammation of the vagina, vulva, and outer urinary tract due to thinning and shrinking of these tissues. Vulvovaginal atrophy also may cause a decrease in lubrication in the vulvovaginal area. As a result, women experiencing vulvovaginal atrophy may not only suffer from decreased sexual enjoyment and day-to-day discomfort due to the lack of lubrication in the vulvovaginal area, but also discomfort during urination and urinary incontinence.

Factors that are known to contribute to vulvovaginal atrophy include menopause, treatments for breast cancer including chemotherapy and for some women, breastfeeding. In all of these causes, a change in the estrogen hormone level is a major contributor to vulvovaginal atrophy.

Until recently, there were limited options for women suffering from vulvovaginal atrophy. Vaginal moisturizers and lubricants only offer temporary relief and often do not provide enough symptomatic relief. Hormone replacement products, either applied locally or systematically, may also be an option, but involve risk of adverse side effects associated with their use. For example, hormone replacement therapies have common side effects such as nausea, vomiting, bloating, weight changes, and in addition may increase the user's risk of certain cancers and cardiovascular events. Furthermore, these types of hormone-based treatments are not recommended for women with, or who are survivors of, breast, ovarian, or endometrial cancers, and are contraindicated for women with a history of stroke or myocardial infarction because of these risks.

More recently, the drug Osphena^{®}, a selective estrogen-receptor modulator that acts on specific estrogen receptors but is not itself a hormone, has become available. Osphena is a daily pill approved for dyspareunia in postmenopausal women; however, the drug acts like estrogen in the body and is currently not recommended for survivors of breast, ovarian or endometrial cancer due to the risk of cancer recurrence. Furthermore, women taking Osphena have experienced varying effects on improving vaginal dryness and have even experienced adverse side effects such as puffiness and redness on various parts of their bodies, severe hot flashes, and weight gain to name a few.

Also recently introduced is the MonaLisa Touch^{®} from DEKA Medical Lasers. This therapy uses a transvaginal, CO₂ fractional laser to stimulate collagen production in the vaginal tissue over the course of three outpatient procedures. While early data from their first US clinical trial looks promising, the therapy has been slow to gain adoption because of its expense, invasive nature, and lack of multi-year safety data.

There is currently no safe, drug-free and highly effective FDA-approved solution for rejuvenating the thin, dry and inelastic vaginal tissue associated with vulvovaginal atrophy. The devices and methods of the invention described herein have been tested clinically and have shown compelling evidence of safety and efficacy as a treatment for vulvovaginal atrophy in both cancer survivors and pre-, peri-, and post-menopausal women.

WO 2013/184798 A1 discloses a system for modifying the focal depth of an ultrasound treatment. An ultrasound transducer has a fixed focal depth and is configured for the delivery of focused ultrasound energy to a target tissue region under a skin surface for a cosmetic improvement of the skin surface. An acoustic spacer is configured for placement between the transducer and the skin surface. The acoustic spacer is configured to mechanically increase a distance between the transducer and the skin surface. The acoustic spacer comprises a transmitting portion that is acoustically coupled to the transducer and the skin surface and is temporarily adhered to the skin surface.

WO 2015/116512 A1 discloses a method of treating vaginal tissue atrophy in a female subject, the method comprising: engaging an energy delivery element with tissue in or around the subject's vagina; applying energy to the tissue from the energy delivery element; and increasing blood flow to internal vaginal tissue to an increased level above a baseline level of blood flow to the internal vaginal tissue, the increased level of blood flow to the internal vaginal tissue persisting after the applying step ceases.

US 2013/338545 A1 discloses an apparatus comprising a skin-application portion configured to move across skin of a subject. At least one acoustic element is coupled to the skin-application portion and configured to be placed in acoustic contact with the skin, and configured to apply ultrasound energy to the skin.

### SUMMARY

The invention is defined in claim 1. Further aspects and preferred embodiments of the invention are set out in the dependent claims. Any aspects, embodiments and examples of the present disclosure which do not fall under the scope of the appended claims do not form part of the invention and are merely provided for illustrative purposes.

There is disclosed herein a method and apparatus (device) which find utility in the treatment of estrogen-deficient women, women with vaginal atrophy (VA), vulvo-vaginal atrophy (VVA), vaginal stenosis, and/or continual discomfort. The method and apparatus locally introduces energy to the genital tissue via ultrasound to rejuvenate tissue in and around the vulvovaginal area. The method and apparatus includes the application of ultrasound from an external energy source to the vulvovaginal tissue, which is acoustically coupled via an acoustic coupler to the vulva and external genitalia of a woman. The method and apparatus serve to do any one or a combination of increase blood flow, increase lubrication to the genital area, reduce dryness, and improve and/or prevent deterioration of vaginal tissue health following repeated use of the apparatus. The device may be either wearable (hands-free) or handheld.

The device can comprise a coupling assembly configured to couple the energy delivery element to the handheld device. In some embodiments, the coupling assembly comprises magnets arranged and configured for magnetically coupling to corresponding magnets on a portion of the energy delivery element. The coupling assembly can comprise any one or more of a hook and loop coupling assembly, snaps, straps, and tabs configured to engage open slots.

The coupling pad can comprise a coupling medium. In some embodiments, the coupling medium comprises a hydrogel. The coupling medium can comprise one or more of agarose, silicone, or water. In some embodiments, the coupling medium comprises gel, foam, oil, liquid, or a combination thereof. In some embodiments, the coupling medium comprises a bacteriostatic additive.

The coupling pad can have a thickness of 3-5 mm. In some embodiments, the coupling pad has a thickness of 4 mm. The coupling pad can have an ovular shape. In some embodiments, the coupling pad has a length of about 35-45 mm. In some embodiments, the coupling pad has a width of about 25-35 mm.

The energy source can comprise an ultrasound generator adapted to provide ultrasound energy at a frequency of about .5 MHz to 2 MHz. In some embodiments, the energy source comprises an ultrasound generator adapted to provide ultrasound energy at a frequency of about 1 MHz. The energy source can comprise an ultrasound generator configured to provide ultrasound energy at a duty cycle of about 20-80%. In some embodiments, the energy source comprises an ultrasound generator configured to provide ultrasound energy at a duty cycle of about 50%. The energy source can comprise an ultrasound generator configured to provide ultrasound energy at a period of 2 to 10 minutes, every day, multiple times a day, every few days, once a week, once every couple of weeks, or once a month. In some embodiments, the energy source comprises an ultrasound generator configured to provide ultrasound energy at an intensity of about 1.0-2.2 W/cm2.

The energy delivery element can comprise a support ring supporting the coupling pad. In some embodiments, the support ring comprises an attachment means to attach the energy delivery element to the handheld device. The attachment means can comprise magnets arranged and configured for magnetically coupling to corresponding magnets on the handheld device. In some embodiments, the attachment means comprises tabs configured to engage open slots on the handheld device.

The energy source can comprise an ultrasound transducer comprising ceramic piezoelectric crystal. In some embodiments, the energy source has an effective radiating area (ERA) of about 2-8 cm2.

The handle can comprise controls configured for activating the device. The device can comprise a recharging assembly.

In some embodiments, the device comprises a feedback mechanism configured for alerting the subject of insufficient contact between the energy delivery element and the tissue in or around the subject's vagina and external genitalia. The feedback mechanism can comprise a strain gauge configured to indicate to the user whether sufficient pressure is being applied to the device. The device can comprise a feedback mechanism configured for alerting the subject of insufficient contact between the energy delivery element and the energy source. In some embodiments, the device comprises a sensor configured to measure a physiological parameter of tissue in or around the subject's vagina and external genitalia relating to vaginal rejuvenation when the energy delivery element is engaged with tissue in or around the subject's vagina and external genitalia, the device being further configured to use information from the sensor to control energy delivery from the energy delivery element. The physiological parameter can be a change in temperature of the issue in or around the subject's vagina, vaginal lubrication, vaginal impedance, vaginal pH, or vaginal tissue elasticity. In some embodiments, the coupling pad is adapted to enhance coupling of the energy delivery element with the tissue in or around the subject's vagina and external genitalia.

There is disclosed herein a method of rejuvenating vulvovaginal tissue in a subject. The method comprises engaging an energy delivery element of a handheld device with tissue in or around the subject's vagina, the energy delivery element comprising a coupling pad detachably connected to the energy delivery element and comprising a dome shaped contour; applying ultrasound energy to the tissue from the energy delivery element; and affecting a measureable parameter associated with vaginal rejuvenation such that the measurable parameter indicates an improvement in vaginal lubrication or vulvovaginal health after the application of ultrasound energy.

In some methods, the coupling pad comprises a coupling medium. The coupling medium can comprise agarose. In some methods, the coupling pad comprises silicone or water.

In some methods, applying ultrasound energy comprises applying ultrasound energy at a frequency of about .5-2 MHz. Applying ultrasound energy can comprise applying ultrasound energy at a frequency of about 1 MHz. In some methods, applying ultrasound energy comprises applying ultrasound energy at a duty cycle of about 20-80%. Applying ultrasound energy can comprise applying ultrasound energy at a duty cycle of about 50%. In some methods, applying ultrasound energy comprises applying ultrasound energy every day, multiple times a day, every few days, once a week, once every couple of weeks, or once a month. Applying ultrasound energy can comprise applying ultrasound energy at an intensity of about 1.5-2 W/cm2.

In some examples, the method comprises removably attaching the energy delivery element to the handheld device. Removably attaching the energy delivery element to the handheld device can comprise engaging magnets positioned on the energy delivery element with magnets positioned on the handheld device. In some methods , applying ultrasound energy is performed by activating a control on a handle of the handheld device. In some methods, engaging an energy delivery element of a handheld device with tissue in or around the subject's vagina comprises engaging an energy delivery element of the handheld device with the patient's introitus.

The method can comprise alerting the user to insufficient contact between the energy delivery element and the subject's tissue. In some examples, the method comprises alerting the user to insufficient contact between the energy delivery element and an energy source. Alerting the user can comprise vibrating the handheld device. In some examples, the method comprises measuring the physiological parameter of the subject's tissue in or around the subject's vagina and controlling ultrasound energy delivery from the energy delivery element based on the measured physiological parameter. The physiological parameter can be temperature, blood flow, vaginal lubrication, vaginal pH, or vaginal elasticity. In some methods, the measurable parameter indicates an improvement in vaginal lubrication after the application of ultrasound energy. The method can comprise alerting the user to sufficient contact between the coupling pad and the energy source via feature locks that provide snap sounds. In some examples, the method comprises adjusting the position of the device in response to an alert indicating insufficient contact between the device and the patient's tissue.

There is also disclosed herein a further device for rejuvenating vulvovaginal tissue in a subject for use with an ultrasound transducer. The further device comprises an energy delivery element comprising a coupling pad configured to engage tissue in or around the subject's vagina and external genitalia, the coupling pad positioned within a coupling pad holder, a top surface of the coupling pad protruding from a top surface of the holder, a bottom surface of the holder comprising an opening configured to receive an ultrasound transducer and acoustically couple the ultrasound transducer to a bottom surface of the coupling pad; and a strap configured to attached the energy delivery element to the ultrasound transducer.

In some examples, a top surface of the coupling pad has chamfered edges. The coupling pad holder can comprise a top portion and bottom portion. In some examples, the top portion and bottom portion are configured to be attached. The top portion and bottom portion can be attached using magnets. In some examples, the coupling pad holder further comprising knobs configured to attach to the strap.

There is also disclosed herein a further method of rejuvenating vulvovaginal tissue in a subject. The method comprises strapping an energy delivery element to an ultrasound device, the energy delivery element comprising a coupling pad positioned within a coupling pad holder, a top surface of the coupling pad protruding from a top surface of the holder, a bottom surface of the holder comprising an opening configured to receive an ultrasound transducer and acoustically couple the ultrasound transducer to a bottom surface of the coupling pad; engaging the energy delivery element with vulvovaginal tissue; applying ultrasound energy to the tissue from the energy delivery element; and affecting a measureable parameter associated with vulvovaginal rejuvenation such that the measurable parameter indicates an improvement in vaginal lubrication or vulvovaginal health after the application of ultrasound energy.

In some methods, applying ultrasound energy comprises applying ultrasound energy at a frequency of about .5-2 MHz. In some methods, applying ultrasound energy comprises applying ultrasound energy at a frequency of about 1 MHz. In some methods, applying ultrasound energy comprises applying ultrasound energy at a duty cycle of about 20-80%. In some methods , applying ultrasound energy comprises applying ultrasound energy at a duty cycle of about 50%. In some methods, applying ultrasound energy comprises applying ultrasound energy at a period of about 8 minutes. In some methods, applying ultrasound energy comprises applying ultrasound energy every day, multiple times a day, every few days, once a week, once every couple of weeks, or once a month. In some methods, applying ultrasound energy comprises applying ultrasound energy at an intensity of about 1.5-2 W/cm2.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIGS.1A - 1C show drawings of a handheld ultrasound device. FIG. 1A and 1B are side views of two possible embodiments of the handheld ultrasound device. FIG. 1C is a drawing depicting a front view of the handheld ultrasound device.
FIGS. 2A - 2C show three different possible embodiments for the handheld ultrasound device.
FIGS. 3A - 3D show variations of acoustic coupler.
FIG. 4A and 4B show a side and front view of yet another example of a handheld ultrasound device.
FIG. 5 is an illustration of placement location and orientation of a handheld embodiment.
FIG. 6 is an illustration of placement location and orientation of a handheld embodiment including direction of ultrasonic wave propagation in relation to the vaginal canal (side view).
FIG. 7 is a diagram showing a closed loop therapy algorithm.
FIGS. 8A and 8B are illustrations of two embodiments of a charging station for the handheld embodiment of the device.
FIG. 9 is a bar graph showing clinical trial data showing an increase in the vaginal blood flow after treatments via the method and device described, herein.
FIGS. 10A and 10B show another example of a handheld ultrasound device.
FIG. 11 illustrates perspective views of various embodiments of coupling pads.
FIG. 12 shows side views of various embodiments of coupling pads.
FIGS. 13A-13D depict Schlieren images for various embodiments of coupling pads.
FIGS. 14A-14D also show Schlieren images for more embodiments of coupling pads.
FIGS. 15A-15C also illustrate Schlieren images for more embodiments of coupling pads.
FIG. 16 depicts results of hydrophone testing for an embodiment of a coupling pad.
FIG. 17 also shows a computational model fit to experimental data from hydrophone testing for an embodiment of a coupling pad.
FIG. 18 shows more results of hydrophone testing for an embodiment of a coupling pad.
FIGS. 19A-19E show images from temperature testing using tissue surrogates.
FIG. 20 illustrates results from the temperature testing of FIGS. 19A-19E.
FIG. 21 depicts computational modeling of various ultrasound transducer sizes.
FIG. 22 shows a meniscus formed during curing of an embodiment of a coupling pad.
FIG. 23 depicts an embodiment of a coupling pad component.
FIG. 24 illustrates an embodiment of an embodiment of a mold for forming a coupling pad.
FIG. 25 shows an embodiment of a coupling pad component.
FIG. 26 depicts an embodiment of a mold for forming a coupling pad.
FIG. 27 illustrates an embodiment of a coupling pad component.
FIGS. 28A and 28B show various views of a coupling pad component.
FIG. 29 depicts part of an embodiment of a mold for forming a coupling pad.
FIG. 30 illustrates an embodiment of a mold for forming a coupling pad.
FIGS. 31A-31C show various embodiments of coupling pad components.
FIG. 32 depicts an embodiment of coupling pad component packaging.
FIG. 33 illustrates another embodiment of an ultrasound device.
FIGS. 34A-34E depicts various views of an embodiment of a main device portion of an ultrasound device.
FIGS. 35A-35I show various views of an embodiment of a support ring of a coupling pad component.
FIGS. 36A-36D illustrate various views of an embodiment of a coupling pad component.
FIG. 37 depicts an embodiment of an ultrasound device.
FIG. 38 shows an embodiment of a coupling pad component.
FIGS. 39A and 39B show another embodiment of a coupling pad component.
FIGS. 40A and 40B illustrate further embodiments of a coupling pad component.
FIGS. 41A and 41B depict embodiments of coupling pad component packaging.
FIGS. 42A and 42B show embodiments of coupling pad component lubrication.
FIG. 43 illustrates another embodiment of a coupling pad component.
FIGS. 44A-44D depicts various views of an embodiment of a coupling pad.
FIGS. 45A-45E show various views of an embodiment of a top portion of a coupling pad holder.
FIGS. 46-46E illustrate various views of an embodiment of a bottom portion of a coupling pad holder.
FIG. 47 depicts an embodiment of an assembled coupling pad component.
FIG. 48 shows an embodiment of a coupling pad component attached to an ultrasound transducer device.
FIGS. 49A-49D are bar graphs showing results from clinical trials showing the effectiveness of an ultrasound device as described herein.

### DETAILED DESCRIPTION

The disclosure provides devices and methods that may be used to promote rejuvenation of a women's vulvovaginal area. The term "vulvovaginal rejuvenation" used herein refers to improving the overall function of the vulvovaginal area that may have suffered from decrease in lubrication, loss of elasticity and resilience, and/or decreased blood flow. Thus, vulvovaginal rejuvenation can refer to any one or a combination of alleviating vaginal dryness, increasing vaginal lubrication, increasing elasticity and/or resilience, and increasing blood flow.

### Handheld ultrasound devices

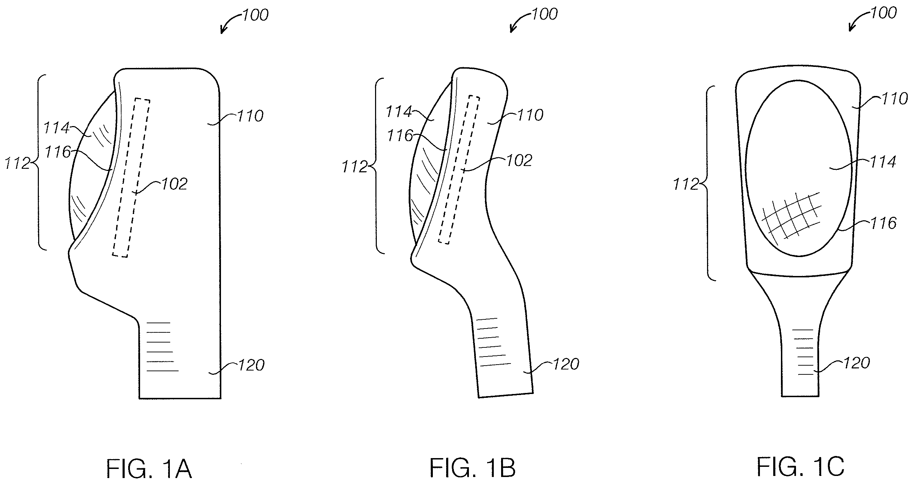

In general, the devices described herein are handheld ultrasound devices that provide ultrasound energy. Ultrasound energy is a form of energy that is created by vibrating or moving particles within a medium, where the medium in needed to conduct and propagate its energy. Ultrasound energy is defined by vibrations with a frequency greater than 20 kHz. As shown in FIGS. 1A - 1C, a handheld ultrasound device 100 includes a device body 110 having an energy delivery element 112 and a handle 120. The handheld ultrasound devices disclosed here also include an ultrasound energy source 102 providing ultrasound energy to the energy delivery element. The ultrasound energy source 102 is typically located within the device body 110, but it may alternatively be contained within the energy delivery element 112. In those latter instances, the device body 110 may have a thickness suitable for accommodating the ultrasound energy source 102 and/or circuitry, and the handle 120 may vary in length and circumference, based upon components and circuitry integrated into these elements. In other examples, the circuitry and necessary components may be separate from the handheld ultrasound device but able to electrically couple to the handheld ultrasound device using standard electrical connectors and cords.

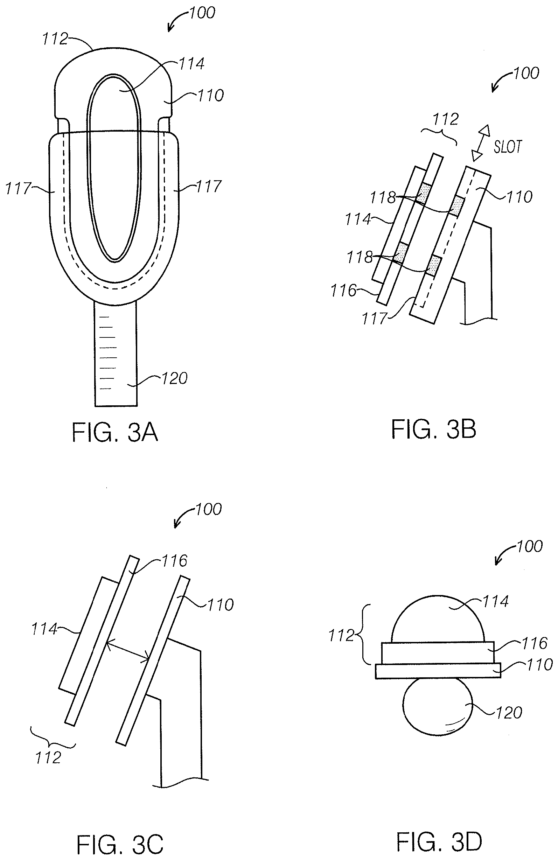

The energy delivery element 112 may include an acoustic coupler 114 or coupling pad and an attachment mechanism 116. Because the acoustic coupler 114 is intended to contact a user's tissue, it is deformable enough to be able to conform readily to the user's anatomy while still having enough structure such that it is able to maintain its overall shape. The acoustic coupler 114 may have a general size and shape that conforms to the female vulvovaginal region (i.e., vulva, labia majora, labia minora, and introitus). The acoustic coupler 114 may be formed of one or more compartments or one or more regions of material or combination of materials. A more detailed discussion on the types of materials useful in forming the acoustic coupler 114 may be found in the sections below. The acoustic coupler 114 may be permanently or releasably attached to the attachment mechanism 116. The acoustic coupler 114 and the attachment mechanism 116 may be mated through any suitable means not limited to hooks and loops, snaps, clasps, magnets, glue, stitching and so forth. The attachment mechanism 116 may be formed of one or more than one layer of semi-rigid material (e.g., foam, rubber) configured to maintain contact with the acoustic coupler 114. The attachment mechanism 116 is also configured to provide acoustic contact with the ultrasound energy source 102 in the device body 110. An example of this is found in FIG. 3B, where the attachment mechanism includes couplers 118 to acoustically couple the energy delivery element 112 with the energy source (not shown) in device body 110. In this example, the device body 110 may also include slots (not shown) that aid with holding the entire energy delivery element 112. In other examples, the attachment mechanism 116 may attach and acoustically couple to the ultrasound energy source 102 through an arrangement including but not limited to a key and slot arrangement, a pin and hole arrangement and so forth.

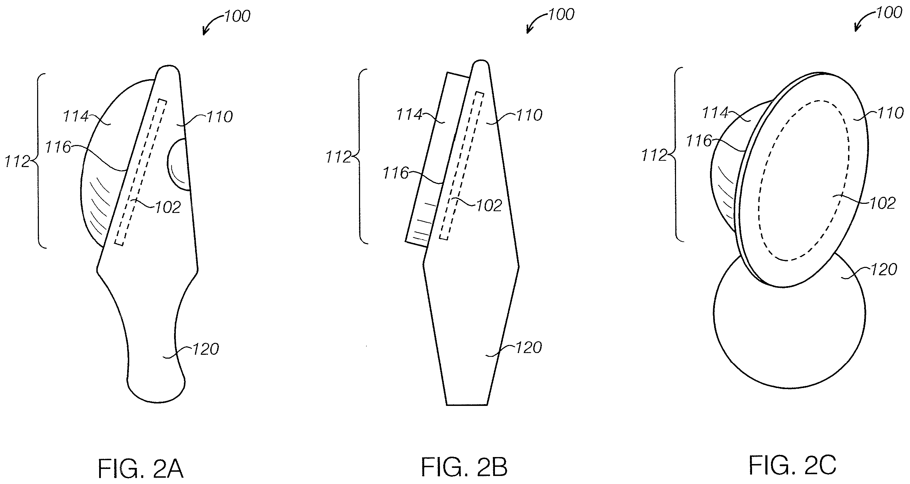

Variations on the handheld ultrasound device are shown in FIGS. 2A-2C. The handheld ultrasound device shown in FIG. 2A includes an acoustic coupler 114 of the energy delivery element 112 that is convex in shape. In another variation, the acoustic coupler 114 of the energy delivery element 112 shown in FIG. 2B has largely uniform thickness throughout. In both of these variations, the acoustic coupler 114 of the energy delivery element 112 may be deformable such that when it is pressed against the user's external genitalia, it is able to more easily conform to the changing topography of the external genitalia tissue. In some variations, the acoustic coupler 114 may be shaped to conform to the general outer vaginal structure. FIG. 2C shows another variation of the handheld ultrasound device where the handle has a semispherical shape. In some other variations, no actual handle is present and the user would hold onto the device body 110 itself instead of holding onto a handle.

The ultrasound energy source 102 may be a piezoelectric (PZT) ceramic or electromagnetic transducer and wave generator disposed within device 100 and in acoustic communication with the acoustic coupler 114 of energy delivery element 112. The PZT may be constructed from piezoelectric materials such as lead zirconate titanate, potassium niobate, sodium tungstate, etc. The transducer assembly may consist of either one or an array of piezo-ceramic ultrasound transducers. The transducer assembly may also be Capacitive Micro-machined Ultrasound Transducers (CMUT) to appropriately apply diffuse ultrasound or provide constructive ultrasound wave interference and focus the ultrasound energy to the target tissue and appropriate vascular bed. The target of the ultrasound energy (unfocused or focused) may be further tuned to cover the mucosal layers of the vaginal canal. In some instances, the ultrasound transducer may have an effective radiating area between 0.1 cm² and 10 cm². In some instances, the effective radiating area may follow the general outline of the outer female genitalia. In some embodiments, the ultrasound may be preferentially focused on the introitus and/or vestibule only, the bottom third of the vagina, the bottom half of the vagina, or cover the entire vaginal canal.

The energy delivery element 112 of the handheld ultrasound delivery device 100 is configured to engage tissue around the subject's vagina as well as the outer genitalia. As mentioned earlier, the energy delivery element 112 may include an acoustic coupler 114 or coupling pad that aids with delivering ultrasound energy to the tissue. The acoustic coupler 114 may be in the form of a preformed gel (e.g., polyethylene glycol-based polymer hydrogel, agar, pectin, carrageenan, etc.), malleable solid or porous pad (e.g., silicone rubber, low durometer polymer, fabrics or flexible foams) or acoustic conducting gel (e.g., ultrasound gel) or fluid-filled bag or compartments, wherein the fluid is water (e.g., deionized, distilled), oil (e.g., mineral), gel, gelatin or other sonolucent and biocompatible fluid. The bag or compartments may be constructed of silicone rubber, low durometer polymer such as poly tetra fluoroethylene (PTFE), Nylon, Latex, low or high density polyethylene (LDPE, HDPE), nitrile, polyisoprene, polyurethane, or urethane; a fabric or a natural (organic) material such as animal skin, agar, pectin of carrageenan. In some instances, acoustic coupler 114 may be biocompatible, non-allergenic, bacteriostatic, and/or antimicrobial. In general, the bag or compartment walls of the acoustic coupler 114 are approximately 0.001 mm to approximately 10 mm in thickness.

The acoustic medium within the acoustic coupler 114 or coupling pad is able to transmit, with minimal loss of acoustic power, ultrasonic energy from the surface of the ultrasonic energy transducer to the target tissue of one or more of the vaginal vestibule, vaginal canal, introitus, vulva, labia minora, labia majora, clitoris, or surrounding area to the genitalia (e.g., perineum, rectum, etc.). The acoustic coupler 114 may also function to collimate and/or focus ultrasonic energy from the ultrasonic energy transducer surface to specific targeted regions within the vaginal canal, introitus, vulva, and/or external genitalia regions. The malleability of the acoustic coupler 114 allows it to fill the spaces of air between the transducer and user's variably shaped genital tissues, but it does not extend beyond the introitus and hence does not penetrate the vaginal canal. In some embodiments, it may penetrate the vaginal canal. The acoustic coupler 114 may act as a safety feature by preventing the occurrence of hot spots from converging waves of incident and reflected ultrasound energy (i.e., standing waves), which may otherwise occur at the interface between the surface of the ultrasound transducer and the genital tissue. It may also prevent surface heating and pain due to inadequate coupling (acoustic impedance mismatch) between the device and the user's tissue. The acoustic coupler 114 may also control the feedback to an open- or closed-loop treatment and/or safety algorithm.

The acoustic coupler 114 or coupling pad may be covered with acoustic coupling gel where it interfaces with the person's tissue, which will most often be the vulva and/or introitus. The gel layer may be pre-applied to the acoustic coupler 114 at the time of manufacture and require the removal of a covering strip or protective, containing layer upon use. The gel layer may also be applied by the user at time of use.

The acoustic coupler 114 or coupling pad may be convex in its profile and elliptical, ovoid or otherwise shaped for roughly conforming to the shape of the vulva and introitus of the vagina (FIGS. 1A, 1B, 2A, 2B, and 2C). The shaped contours of the acoustic coupler 114 may also function to focus the ultrasonic energy to the target areas within the vaginal canal, vascular bed, or more superficially to the external genitalia. The shaped contours of the acoustic coupler 114 may also function to provide tactile or contact feedback to the user so that the user knows the device is being held in the correct location and position. The acoustic coupler 114 may be between 1 mm and 50 mm in thickness or 1- 5 mm, 1 - 10 mm, 1-20 mm, 25 - 50 mm, between 10 mm and 200 mm in length, 10 - 20 mm, 15 - 50 mm, 50 - 75 mm, 75 - 150 mm, 100 - 150 mm, 150 - 200 mm and between 10mm and 50mm in width, 10- 20 mm, 20 - 40 mm, or 25 - 50 mm. In some variations, the acoustic coupler 114 may be approximately 10 mm to approximately 200 mm in length and approximately 10 mm to approximately 50 mm in width. The acoustic coupler 114 may be one size, have multiple size options or may be custom fit to each user. The orientation of the acoustic coupler 114 relative to the target tissue areas may also be adjusted by the user based on her anatomy, with feedback from the system.

The acoustic coupler 114 may be reusable and capable of being disinfected after use. Alternatively, the acoustic coupler 114 or coupling pad may be disposable and discarded after each individual use or several uses of the device. FIG. 3A and 3B show one example of how the energy delivery element 112 (and its acoustic coupler 114) may be detachably connected to the device body 110. In this example, the device body 110 includes side slots 117 into which the sides of the attachment mechanism 116 may slide into and couple to the device body 110.

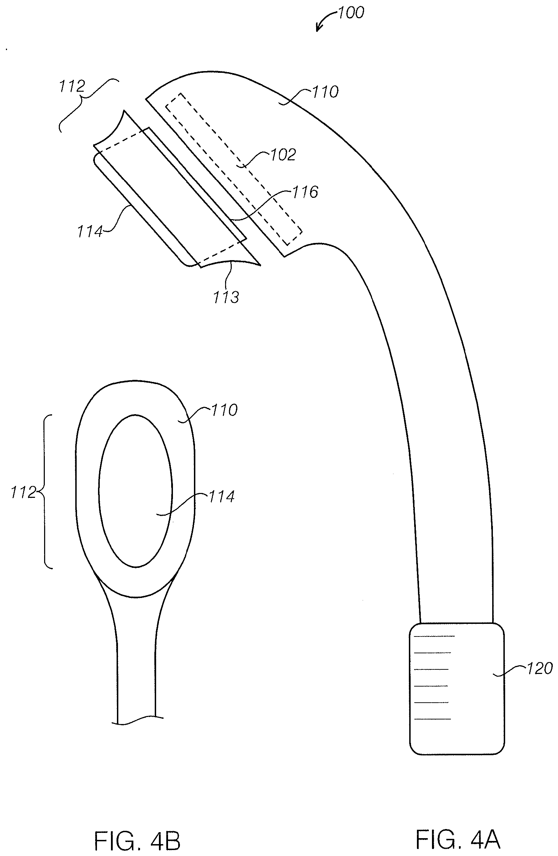

FIGS. 4A and 4B show another variation of the energy delivery element 112 where a rim 113 further aids with maintaining coupling of the energy delivery element 112 with the device body 110. The rim 113 may connect to the device body 110 via corresponding threads, snaps, adhesives, clasps, magnets, buttons, and so forth.

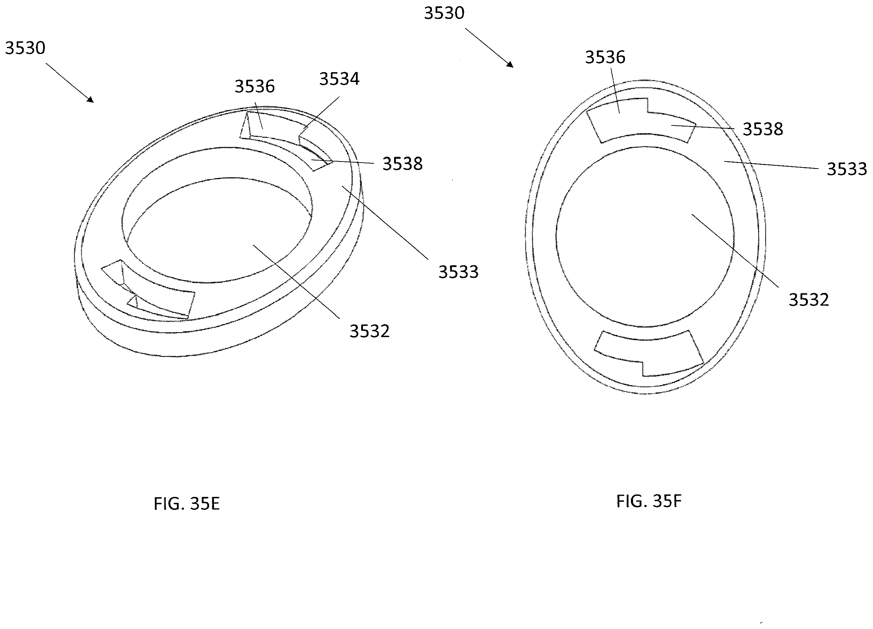

In the case where only the acoustic coupler 114 portion of the energy delivery element 112 may be disposable, the energy delivery element 112 may be attached to the device body 110 in a multitude of ways. The coupling mechanism may include, but is not limited, to an open slot feature whereby the acoustic coupler 114 may slide into place (e.g., as shown in FIGS. 35E-35I), a hinged or movable clasping feature that secures the acoustic coupler 114 into place, an adhesive area on which the acoustic coupler 114 may be affixed, an internally threaded feature into which the acoustic coupler 114 may be threaded down into contact with the attachment mechanism 116, or some geometric feature on the distal end of the handle that secures the acoustic coupler 114 to the surface of the attachment mechanism 116. In some instances, the acoustic coupler 114 may be permanently attached to an attachment mechanism 116 which then may mate with the device body 110 through corresponding magnets arranged about the energy delivery element and the device body 110. Additionally, the acoustic coupler 114 may be connected to the attachment mechanism 116 via elastic or Velcro^{®} straps, by features on the reusable portion of the device that penetrate into the coupling feature medium (e.g., pins, barbs or hooks), or by vacuum or suction attachment.

The relative orientation of the ultrasound energy source 102 and the energy delivery element 112 may be achieved using couplers 118 that mate between the ultrasound energy source 102 and the attachment mechanism 116 (see FIG. 3B). More focused ultrasound output may be achieved through arranging the ultrasound energy source 102 in a particular orientation or through automatic adjustments to the ultrasound energy source 102 based on closed-loop feedback during use.

As mentioned above, the device has features that provide feedback to the user to inform whether or not contact between the transducer face and the coupling pad or the contact between the coupling pad and the user's tissue is non-optimal (for safety to the user, for the integrity of the device, and for efficacy of treatment). These feedback mechanisms include simple feature locks that may provide "snap" sounds to inform the user the part is seated; pressure, impedance or other sensors between the transducer and the coupling pad that provide direct feedback to the user; or alarms (e.g., vibrating alarm) on the ultrasound generator that are based on sensor feedback. Such feedback can reflect inadequate and/or unsafe coupling between the transducer and the acoustic coupler 114, or inadequate and/or unsafe coupling between the acoustic coupler 114 and the user's tissue. The device may have a closed-loop algorithm to automatically shut off ultrasound delivery if unsafe coupling has been detected for a period of time.

The feedback may be based on reflected ultrasound energy or on some other parameter, such as tissue temperature measured by a temperature sensor. The contact feedback information can be displayed to assist in adjustment of the acoustic coupler 114 to the user's tissue or the attachment mechanism 116 to the device body 110. This display may include blinking lights of different colors similar to a tuning instrument, audible cues, mechanical/vibratory cues, and so forth. The surface-to-surface contact between the transducer face and the acoustic coupler 114, and/or the interface between the acoustic coupler 114 and the user's anatomy may be adjusted just before and/or during ultrasound treatment administration to maintain good acoustic coupling between the surfaces. This adjustment can be achieved by spring-loaded features, magnetic or mechanical snap fits, elastic materials (e.g., silicone or elastic bands) that wrap around the back of the transducer, adhesives, or visual, audio, or other types of cues to alert the user to move the device slightly and/or apply more force herself.

In some instances, the handheld ultrasound device may have several sensors embedded within the acoustic coupler 114 that allows for measurement of various physiologic parameters. Physiological parameters may include mucosal/dermal blood flow, possibly measured with Doppler ultrasound, Doppler laser imaging, temperature measurement (thermometer), infrared imaging, thermography, or photoplethysmography. Parameters may include vaginal lubrication measured for example utilizing humidity sensors, absorbent materials, or other methods for detecting lubrication and/or secretion. Parameters may include tissue temperature, measured for example utilizing thermometers or thermocouples, or other methods for detecting temperature changes. Additional physiologic parameters relating to vulvovaginal health and sexual function may also be measured utilizing the appropriate sensor setup. Parameters may include tissue impedance and other various markers of vulvovaginal tissue health such as: tissue elasticity, type and amount of vulvovaginal fluid present, vulvovaginal pH, friability of vulvovaginal mucosa, amount of vaginal moisture present, degree of inflammation present, and percentage of parabasal, intermediate, and/or superficial squamous cell types present in the vulvovaginal epithelium. Parameters may also include cellular calcium uptake, cellular activity and metabolism, protein synthesis by fibroblasts, collagen synthesis and deposition, cell proliferation, cell degranulation, synthesis of non-collagenous protein (NCP), production and signaling of Vascular Endothelial Growth Factor (VEGF), formation of endothelial cells, release of endothelial growth factors, angiogenesis, release of angiogenesis-related chemokines or cytokines (e.g., Interleukin 8, IL-8, or basic Fibroblast Growth Factor, bFGF, or TNF-alpha). The parameters measured may also include biomarkers of negative side effects of ultrasound treatment, such as markers of inflammation and histamine production.

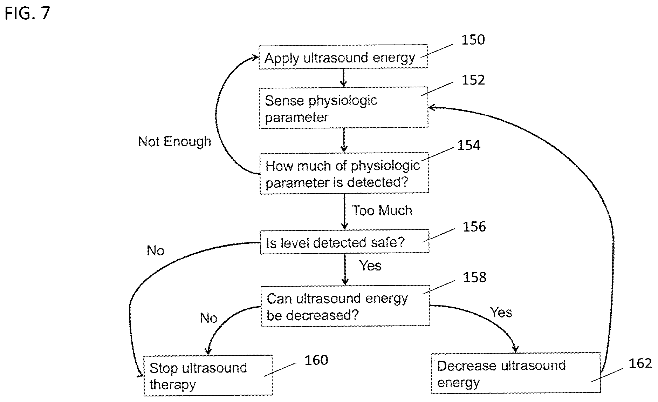

The sensors embedded within the device may allow for closed-loop feedback control of ultrasound application, as shown by the flow chart shown in FIG. 7. In general, the feedback control will be able to sense a physiological parameter 152 after ultrasound energy is applied 150. Once the physiological parameter is detected 154, the algorithm will query whether the detected physiological parameter level is safe 156. If the detected physiological parameter level is not safe, then the ultrasound therapy stops 160. If the ultrasound level is at an unsafe level, the algorithm will also determine if the ultrasound energy may be decreased 158. If the ultrasound energy cannot be decreased, the algorithm will halt the ultrasound therapy 160. If a decrease in ultrasound energy is possible 162, the device 100 controls will decrease the ultrasound energy output. At a later time during the ultrasound therapy, the algorithm will again sense the physiological control to determine if the ultrasound energy output is at a safe level. On the other hand, if the physiological parameter detected is too low or below a threshold value, the controls may send signals to the transducer to increase ultrasound energy output.

In one example, vulvar tissue temperature may be measured by a sensor in the coupling pad. If the temperature rises to a level that could potentially cause damage to the user, the feedback loop automatically adjusts the energy delivery parameters or turns off the energy delivery altogether. In another example, the device increases energy delivery if the temperature of the user's vaginal or external genitalia tissue is not high enough. In another example, the device measures physiologic outcome parameters (e.g., vaginal blood flow and/or lubrication) and automatically increases or decreases ultrasound delivery to achieve the desired outcome (e.g., more or less vaginal blood flow and/or lubrication). In another example, the device may monitor for adverse treatment effects and automatically titrates the ultrasound energy delivery to minimize side effects while still achieving the desired treatment outcome.

In general, the handle 120 allows the user to comfortably position and maintain the ultrasound device against her vaginal or external genitalia tissue. In some variations, the handle 120 of the handheld ultrasound device may also include a variety of components. In some instances, the handle 120 may include an ultrasonic wave generator, an ultrasound transducer, accompanying electronics, or any combination of these elements inside. Alternatively, the handle 120 may merely support the structure that generates and delivers ultrasound treatment, but lacks any other parts required for ultrasound generation and ultrasound treatment administration. In the latter case, the handle would physically connect to the ultrasound transducer, and also be connected via a cord or wirelessly to another device that houses the electronics needed to supply the power and generate the ultrasound energy through the ultrasound transducer. In yet other examples, the handle may include a display for showing various parameters such as session time, adequate/inadequate contact, and so forth.

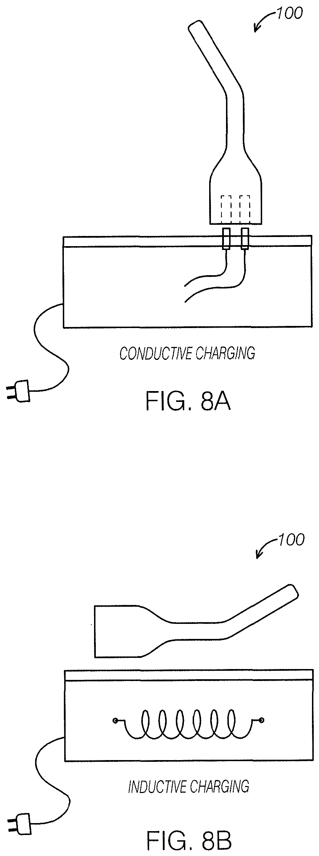

The handheld ultrasound device may also include a power source that is rechargeable and can be recharged with an external recharging station, or that is disposable and consists of replaceable lithium-ion or other sources of direct current (i.e., batteries). The device may also be powered by alternating current from an external source (e.g., an electrical outlet). Where the device is rechargeable, the recharging station may physically couple to the ultrasound handle. A portable recharging component may be coupled to the handle through corresponding geometric features on the handle and the recharging component. (FIG. 8A). The recharging station may be electronically coupled to the rechargeable device via conductive recharging pins in the handheld and corresponding pins in the recharging station, or by proximity via inductance between the device and recharging station (FIG. 8B). The rechargeable transducer handle ultrasound device may hold a charge allowing for one or more treatments before requiring recharging. As alluded to earlier, the rechargeable transducer or ultrasound device may also include feedback for alerting the user as to when the treatment duration is finished and/or when there is too high a temperature at the ultrasound transducer surface (for safety) including but not limited to visual indicators (light emitting diodes or electric lamps), vibratory motors which pulse for a short duration of time or auditory or vibratory signals. Additional signals may also be provided to notify the user that the handheld ultrasound device requires recharging.

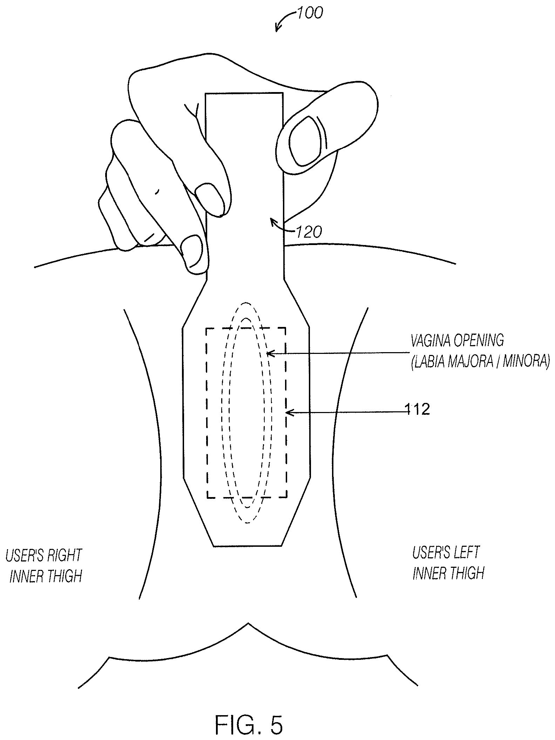

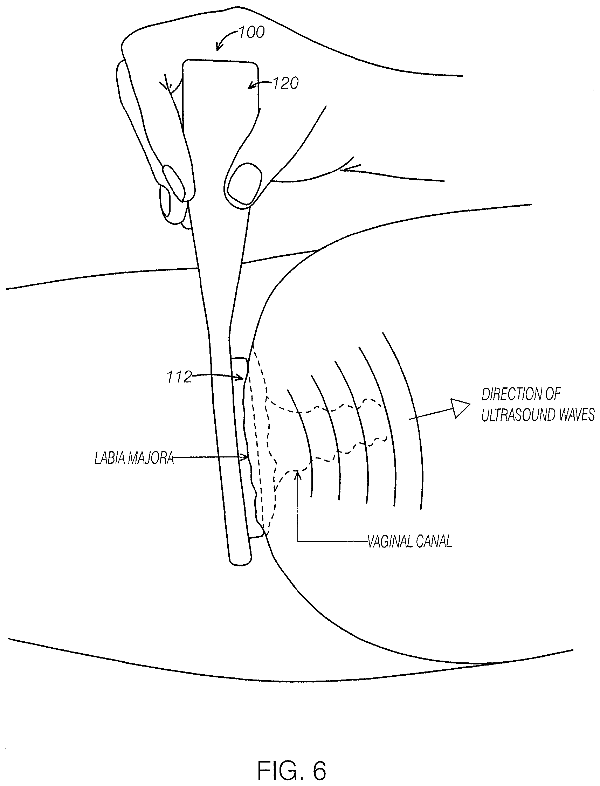

The device body 110 of the handheld ultrasound device may be any suitable size and shape. In some instances, the device body 110 may be elliptical in shape (FIGS. 1C, 3A, and 4B). In other examples, the handheld ultrasound device may have a more elongated shape such that the user may grip the handle (FIGS. 5 and 6). In some examples, the handheld ultrasound device may not include a handle, and where the device rests in the palm of the user's hand. In other examples, the ultrasound device may attach to (as opposed to being held by) the user's hand such as in a glove-based device that may be worn by the user, and where the ultrasound components are encompassed within the glove (e.g., palm portion). In yet other examples, the ultrasound device may be a ring-type design that may be worn on one or multiple fingers.

In general, the handheld ultrasound device may be used at home or in a clinical setting. If used at home, the user may be able to apply the ultrasound treatment herself. The device may be designed in such a way to be conducive to one-hand self-application (FIG. 5). Alternatively, the device and ultrasound treatment may be administered by a partner. In other embodiments, the device may be entirely hands-free, while still maintaining proper tissue contact, orientation, and treatment efficacy. If used in a clinic setting, the device and ultrasound treatment may be administered by a trained professional. Or in some instances, the user may be able to treat oneself after being trained by a professional.

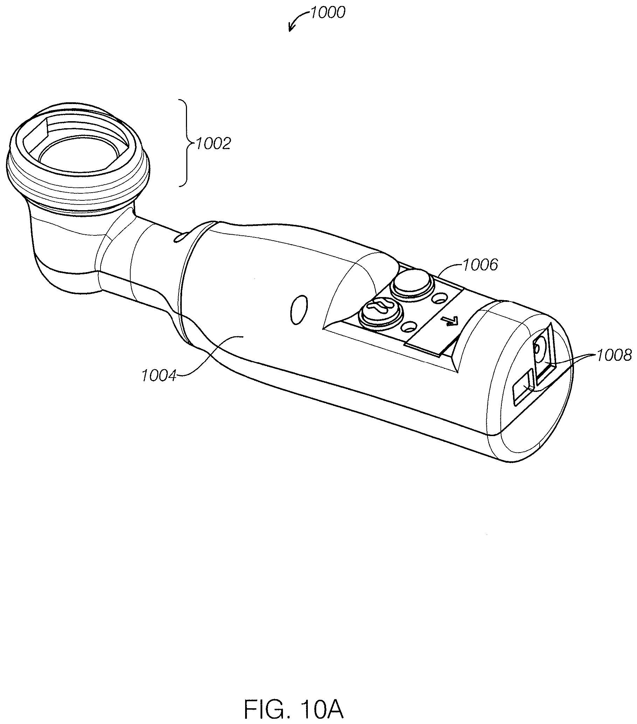

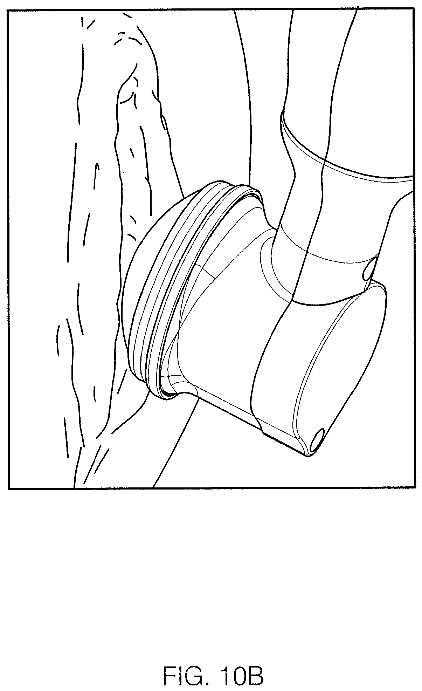

FIG. 10A shows another example of the handheld ultrasound device 1000 previously shown and described. As can be seen, this handheld ultrasound device includes a disposable coupling pad 1002 configured to couple to a woman's vulvovaginal region and an ultrasound device 1004 configured to deliver energy to the coupling pad 1002. The handheld device includes electronic components for generating the ultrasound energy to be delivered through the coupling pad. In this example of the handheld ultrasound device, the controls 1006 for the handheld device are located on a lower surface of the device. In other examples, the controls may be on an alternative surface of the handheld device (e.g., along a side surface or on a top surface). In yet other examples, the controls may not all be located on one surface of the device, but instead, may be disposed on multiple surfaces of the handheld device. The handheld device shown in FIG. 10A and in the prior figures may also include connection ports 1008 for recharging the device as well as ports for transferring data from the handheld ultrasound device to other communication devices (e.g., laptop, desktop, smart phone, tablets). In some instances, there may be applications for a smart phone or tablet associated with using and controlling the handheld ultrasound device. FIG. 10B shows the device of FIG. 10A during use, placed at the introitus, between the labia.

### Methods and parameters for using the handheld ultrasound devices

The methods for using the handheld ultrasound devices described herein may be used to improve vulvovaginal and vulvar tissue health. A device of the invention may be used on an as-needed basis, for example, prior to sexual intercourse to increase blood-flow and induce lubrication. The overall health of the vaginal and vulvar tissue may be improved by use of the device multiple (more than one), times a day, daily, weekly, multiple (two, three, four, five, or more than five) times a week, or monthly as a periodic treatment. The actual length of time for each session may be on the order of seconds to tens of minutes. In practice, the ultrasound sessions may be a few minutes to ten minutes. During such sessions, increases in blood flow to vulvovaginal tissue and vulvovaginal lubrication may be measurable. In some aspects of the invention, the device may be used a single time prior to a sexual encounter. In other aspects of the invention, the device may be used repeatedly unrelated to sexual activity. In both regimens, periodic use may revitalize vulvovaginal lubrication and/or tissue and improve vulvovaginal health.

In other instances, methods for using the handheld ultrasound devices may be used as a preventative measure. The output from the handheld ultrasound device may improve mucosal vascularity, restore tissue elasticity, promote angiogenesis, encourage collagen growth/regrowth, improve muscle tone, promote the repair of soft tissue, and/or to increase constitutive lubrication.

The devices and methods described herein for rejuvenating the user's vulvovaginal area and external genitalia may be used in the privacy of her own home although application of the ultrasound therapy may also be performed in a medical office setting.

In general, the handheld ultrasound devices described herein may be placed external to the vagina and locally apply ultrasound energy to all or a portion of the vaginal vestibule, vaginal canal, introitus, vulva, labia minora, labia majora, clitoris, or surrounding area to the genitalia (e.g., perineum, rectum, etc.) as shown in FIGS. 5 and 6. The device may also sit adjacent to the external genitalia, such as in the region of the mons pubis or proximal thighs. The handheld ultrasound device may cover some of the vulva or the clitoris, much like a feminine hygiene pad, but is completely non-penetrating of the vaginal canal. In some embodiments, the device may enter a small portion of the vaginal canal, while also residing outside the vaginal canal. Optionally, the device may be completely encased within the vaginal canal.

In general, the devices described herein are configured to provide ultrasound energy. While typically ultrasound energy may range anywhere between 20 kHz and 20 MHz, the ultrasound energy delivered from the handheld ultrasound device is approximately between 80 kHz and 3 MHz. At the range of 1 MHz and 3 MHz, optimal energy deposition occurs at more shallow tissue depths. In some instances, the user may vary the handheld ultrasound device's output for optimal energy deposition at more shallow tissue depths. The device may include features that provide for optimal energy deposition to all or a portion of the vaginal vestibule, vaginal canal, introitus, vulva, labia minora, labia majora, clitoris, or surrounding area to the genitalia (e.g., perineum, rectum, etc.).

In some instances, the ultrasound energy may be delivered at an intensity range of 0.1 W/cm² to 5.0 W/cm². More practically, the handheld ultrasound device is adapted to provide ultrasound intensity between approximately 0.25 W/cm² to approximately 2.5 W/cm². The intensity of the ultrasound energy is the acoustic ultrasound power over the area of the transducer.

The ultrasound output from the handheld devices may increase the temperature of the tissue being treated. In some instances, the ultrasound output may be designed to heat tissue to a minimum of 37°C, but no greater than 44°C, so as not to cause damage to the target or surrounding tissue. The increase in temperature from 37°C to its upper limit may be increased stepwise or ramped up in a continuous fashion. Where the duty cycle of the ultrasound output is less than 100%, the increase in temperature may be coordinated with when the ultrasound beam is on or off. In other instances, the ultrasound output may be designed not to heat the tissue at all above the average core body temperature of 37°C in order to induce only non-thermal effects in the tissue from ultrasound.

In some instances, the handheld ultrasound device may include an automatic duty cycle adjustment feature. The automatic duty cycle feature may be an open-loop (requiring action by the user) or closed-loop (not requiring action by the user) treatment algorithm. An automatic duty cycle adjustment feature is useful to ensure appropriate overall energy delivery to the tissue while maintaining user safety thereby providing optimal treatment for desired outcome. In some examples, the device may be highly customizable by the user to modify the treatment (e.g., delivery method, duration, and quantity of ultrasonic energy delivered to the vulvovaginal or surrounding tissue).

The handheld ultrasound device may have a duty cycle of anywhere between 20% and 100%. The term duty cycle refers to the percentage of time that a pulsed ultrasound wave is on (e.g., a 50% duty cycle means that a pulsed wave is on 50% of the time). At a duty cycle of 100% (also called a continuous duty cycle), the pulsed wave is on 100% of the time. The intensity and duty cycle can either be individually set for each treatment or set once for all subsequent treatments. In some embodiments, the intensity and duty cycle can be set by a trained physician, the user, or an advocate for the user. The intensity and duty cycle may be automatically set as a feature pre-programmed into the device and may or may not change. In some embodiments, the intensity and duty cycle settings are changed based on previous treatment duration and results.

The methods disclosed herein for using the handheld ultrasound devices may improve one or more indicators of vulvovaginal tissue health including: elasticity, type and amount of vaginal fluid present at rest (unaroused state), type and amount of vaginal fluid present during arousal, vaginal pH, friability of vaginal mucosa, amount of vaginal moisture present, and degree of inflammation. In some instances, the amount of vaginal fluid may also be measured during an aroused state. These parameters may be measured by the Vaginal Health Index (VHI) (e.g., by trained observer, by computer imaging). The device may also improve the distribution of cell types present in the vaginal epithelium, as measured by the Vaginal Maturation Index (VMI) and reflective of the maturity and health of the vaginal epithelium. The cell types measured in this index are parabasal, intermediate, and superficial squamous cells. The methods and devices of the invention may improve the distribution of each of these types of epithelial cells towards a healthier tissue state.

More specifically, the methods associated with the handheld ultrasound devices may result in one or more of the following effects on vulvovaginal tissue: increase cellular calcium uptake, increase cellular activity, increase cell metabolism, increase protein synthesis by fibroblasts, promote collagen synthesis and deposition, promote cell proliferation, promote cell degranulation, increase synthesis of non-collagenous protein (NCP), increase production and signaling of Vascular Endothelial Growth Factor (VEGF), stimulate the formation of endothelial cells, stimulate the release of endothelial growth factors, promote angiogenesis, increase in angiogenesis-related chemokines or cytokines (e.g., Interleukin 8, IL-8, or basic Fibroblast Growth Factor, bFGF, or TNF-alpha).

### Example 1

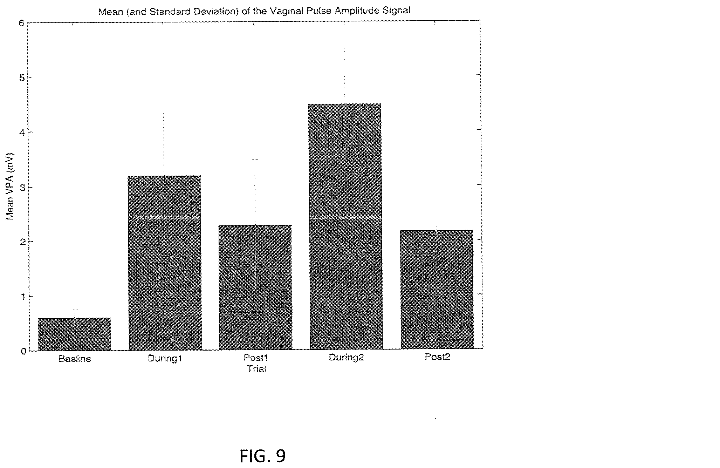

A study was conducted with 9 subjects to evaluate the ultrasound devices and methods of the disclosure. The results indicate that there is a local increase in blood flow and temperature, as described in WO 2015/116512 A1. FIG. 9 shows, for nine patients, the average increase in vaginal blood-flow during and after two subsequent treatments with an embodiment of the device and method described herein.

### Coupling Pad Design Considerations

As described herein, the acoustic coupler or coupling pad is a sonolucent, deformable gel pad that physiologically conforms to the introitus (vaginal opening) and surrounding structures while gently ensuring consistent, safe therapy delivery. In order to ensure safe and effective energy delivery to the appropriate anatomical target, the coupling pad has several performance goals. First, it should ensure solid contact between the ultrasound device and the user's tissue to encourage loss-free ultrasound energy transmission. Second, to achieve intimate, consistent contact with a variety of anatomies, the coupling pad should be deformable. Finally, to maintain safety, the coupling pad can prevent burns by serving as a buffer between the ultrasound transducer and the user's skin.

To determine the ideal coupling pad design, three inter-related parameters were examined: 1) material, 2) size and shape ("Contour"), and 3) overall acoustic properties. Desired coupling pad characteristics were considered both from the perspectives of technical performance and commercialization. From a performance standpoint, the coupling pad should conform comfortably to the anatomy of the patient at the treatment site (introitus), be biocompatible with a mucosal membrane, minimize energy attenuation in order to maximize therapy delivery, and avoid unintended and unsafe beam focusing. From a commercialization standpoint, the coupling pad should be simple and cost-effective when manufactured at scale. The results of this research are summarized here.

### Material Selection

Material selection has the largest effect on therapy efficacy and manufacturing costs and thus was investigated first. Based on experience, observation, and preliminary literature research, four candidate materials were selected based on their lubricity and sonolucent profiles. Lubricity can be key to maximizing energy delivery, as it decreases the acoustical impedance at the tissue interface by minimizing air gaps between the ultrasound device and the user's tissue. The candidate materials were either self-lubricating (hydrogels and agarose) or could easily be lubricated in a secondary manufacturing step (e.g. coated silicone rubber).

Sonolucence is also important to maximize energy delivery, and these materials were selected because they are similar to water (an ideal ultrasound coupling medium). The inventors have found that water as a coupling medium has demonstrated increases in vaginal blood flow and lubrication.

The four candidate materials were scored across five categories: Deformability, Cost of Materials, Biocompatibility, Manufacturability, and (degree of) Ultrasound Attenuation. These categories were weighted based on relative importance, and a total score was calculated to determine which candidate materials should be carried forward for the next stage of testing. Results are presented in the Decision Matrix in Table 1. Agarose and Silicone Rubber were found to be the two best materials. However, as described above and herein, other materials may also be used, in some embodiments.

**Table 1. Material Decision Matrix. Each material was scored on a scale of 1-3 (3=highest), and each category was ranked based on importance (5=most important). Agarose and Silicone resulted in the highest scores and were carried forward to the next stage of testing.**

| | **Hydrogel** | | **Alginate** | | **Argarose** | | **Silicone Rubber** | |
|---|---|---|---|---|---|---|---|---|
| **Category (Rank)** | Score | Rationale | Score | Rationale | Score | Rationale | Score | Rationale |
| Deformability (3) | 3 | Can deform to fit the anatomy | 3 | Extremely deformable | 2 | Fairly deformable depending on agarose concentration. | 2 | Fairly deformable at low durometer (i.e. Shore 10A) |
| Cost of Materials (1) | 1 | High cost of materials, multiple ingredients required, high initial cost to create formulation | 3 | Low cost of materials | 3 | Very low cost, one material required | 2 | Fairly low cost, however medical grade is more expensive |
| Biocompatibility (5) | 1 | The UV-curing component (Irgacure) is a known skin irritant | 2 | Breaks down in the presence of salt (e.g. sweat) | 3 | FDA approved as a diuretic, not a skin irritant | 3 | Used in many biomedical applications. not a skin irritant |
| Manufact -urability (2) | 1 | Disposable molds and specific UV cure equipment required, high initial cost | 1 | Very difficult to create in house, does not hold shape well | 3 | Easy to make, pour, and maintains a shape in a mold. | 3 | Easy to mix, pour, and maintains a shape in a mold. |
| Low Ultrasound Attenuation (4) | 3 | Due to high water content, hydrogels have a low ultrasound attenuation. | 2 | Contains air bubbles and suspended solids which attenuate ultrasound. | 3 | Very low US attenuation. [Validated by results in Wattmeter, Optison, and Hydrophone tests results below.] | 1 | Attenuates the ultrasound. [Validated by results in Wattmeter & Optison tests below.] |

| **Final Weighted Total** | | | | | | | | |
|---|---|---|---|---|---|---|---|---|
| | **23** | | **32** | | **43** | | **33** | |

### Material Formulation Determination

Silicone and agarose were researched in further detail to determine the specific formulations that would meet the coupling pad performance goals of: 1) minimizing ultrasound attenuation, 2) having a high tear strength (durability), and 3) being non-sticky.

A range of silicone durometers were explored. Since silicone is not naturally lubricious, high deformability could be used to meet performance goal #1. Higher durometer silicones (Shore Hardness 30A and higher) were tested but did not deform to the female anatomy in some embodiments. Very low durometer silicones (Shore Hardness 00-20) demonstrated better deformation, but did not meet performance goal #3, as they stuck to hair and skin in initial testing. Silicone with a Shore 10A Hardness provided the right trade off between deformability, durability, and tackiness. It was selected as an optimal formulation.

Agarose concentrations of 0.5 % to 5 % (mass/volume of water) were examined. Agarose is naturally lubricious, and the lower the agarose concentration, the more lubricious the contact surface. Thus all concentrations explored met performance goal #1 and 3; however, the lower agarose concentrations did not meet performance goal #2 in some embodiments. The 2% agarose optimized the performance goals. While 2% agarose was found to be a preferred material, note that other concentrations of agarose (e.g., 0.5%-5%) can also be used.

### Wattmeter Material Testing

Wattmeter tests were conducted to compare the ultrasound transmission characteristics of the selected silicone and agarose formulations. A Wattmeter was used to measure and compare the total acoustic power output through the two candidate materials. Materials with poor ultrasound conduction (high attenuation) will produce lower power output. For testing, each material was placed over a commercially available ultrasound transducer set to a fixed output (Intelect TranSport^{®}, Chattanooga, with settings: 1MHz, 0.8 W/cm², 20% duty cycle).

Shore 10A silicone and 2% agarose were tested, and benchmarked against a bare ultrasound transducer ("No Coupling") as well as a commercially available, ultrasound stand-off (AquaFlex^{®} Ultrasound Ge1 Pad, Parker Laboratories, Inc.). The Aquaflex pads are specifically designed for use with therapeutic ultrasound and are currently being used by in a long-term (chronic) clinical trial. Wattmeter test results are listed in Table 2. Each material (including "No Coupling") was measured 3 times and averaged.

**Table 2. Results of the Wattmeter testing reveal that 2% agarose demonstrates the least amount of ultrasound attenuation. ^Note: 2% agarose is equivalent to "No Coupling," as the reading differences are within the noise of the wattmeter measurements.**

| **Coupling Material** | **Reading 1(Watts)** | **Reading 2 (Watts)** | **Reading 3 (Watts)** | **Average (Watts)** |
|---|---|---|---|---|
| **No Coupling** | 0.65 | 0.75 | 0.70 | 0.70 |
| **AquaFlex Gel Pad** | 0.60 | 0.65 | 0.60 | 0.62 |
| **Silicone, Shore 10A** | 0.35 | 0.30 | 0.45 | 0.36 |
| **2% Agarose** | 0.70^ | 0.75 | 0.75^ | 0.73^ |

Based on the wattmeter testing, it was clear that Shore 10A silicone attenuates ultrasound energy, while 2% agarose did not appear to attenuate ultrasound energy.

### Contour Selection

The final contour determines the level of conformance to the anatomy as well as the ultrasound beam shape and efficacy.

### Size

The size of the contour can be key to ensuring the device will be placed correctly by the user and the therapy will reach the appropriate tissue. The target area of contact is the vaginal opening (introitus) and immediately surrounding tissue. Contact should be avoided with non-target, nearby tissue including: the clitoris, urethra, and anus.

Appropriate contour sizes were determined based on the typical range of vulva measurements. The mean labia minora width is reported as 20 mm on each side, and the mean length from the bottom of the urethra to the start of the perineum is 22-32 mm. [Lloyd 2005, Cao 2014]. Based on these measurements and initial testing with healthy volunteers, the base of the contour was designed to be 27 mm wide and 36 mm long. The contour was also given a taper to its top surface to fit well between the labia minora in a variety of women.

### Fit Testing - Overall Shape

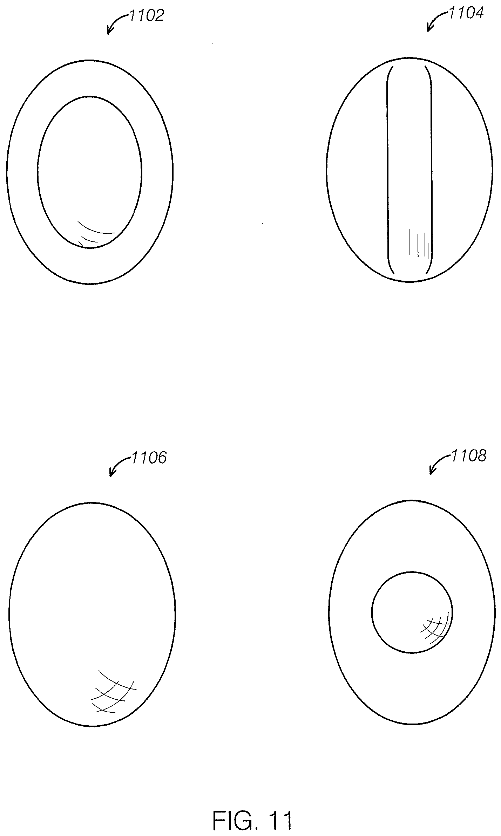

In order to determine the ideal contour, four prototypes were modeled and fit tested on four healthy volunteers (FIG. 11: Oval Nub 1102, Ridge 1104, Dome 1106, and Round Nub 1108). Each met the following objectives:
- Reduce air gaps between the Coupling Pad and the vulva
- Help the user "self-navigate" the device into the correct position
- Fit all labia sizes and shapes ("One size fits all")
- Feel comfortable to the user

The volunteers were given all four contours, instructed on proper placement, and were asked to report feedback based on comfort and ease of placement. The contours were ranked 1 to 4 (1 = best, 4 = worst) by each volunteer, and the average results are presented in Table 3.

**Table 3. Results of the Contour fit test.**

| | **Oval Nub** | **Ridge** | **Dome** | **Round Nub** |
|---|---|---|---|---|
| **Comfort** | 1 | 4 | 2 | 3 |
| **Self-Navigation** | 1 | 4 | 3 | 2 |

All volunteers found the Oval Nub and Dome to be more comfortable than the Round Nub and Ridge. They also all considered the Ridge to be the *least* intuitive to place in the correct position. While the oval nub and dome were found to be more comfortable than the round nub and ridge contour shapes, the round nub and ridge contour shapes can be used, in some embodiments.

### Fit Testing - Contour Height Determination

Contour prototypes were made from both Shore 10A silicone and 2% agarose in the three remaining Contour shapes with varying heights. These shapes and materials were again tested with four healthy volunteers for fit feedback. Bench testing was also completed with an anatomical model to visually examine the contact locations and level of conformance provided by each Contour prototype.

In addition, two independent gynecologists who regularly treat women with VVA were asked to evaluate the shape-height prototypes. Both physicians validated the volunteers' results, confirming that the prototype contours would conform well to a variety of female anatomies.

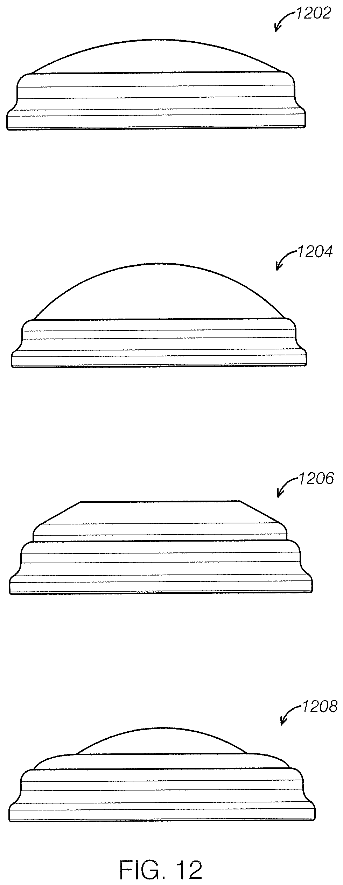

The top three shape-height combinations from the healthy volunteers' feedback were selected for further testing. Fig. 12 shows the contour shapes that were tested further. Coupling pad 1202 ("short dome") comprises a dome shape and a thickness of about 4.5 mm. Coupling pad 1204 ("tall dome") comprises a dome shape and a thickness of about 485 mm. Coupling pad 1206 ("flat top") comprises a dome shape with a top portion of the dome removed, and a thickness of about 6 mm. Coupling pad 1208 ("oval nub") comprises a generally convex, rounded base and an oval shaped convex feature protruding from the rounded base. The oval nub contour 1208 comprises a thickness of about 7 mm.

The coupling pad contours were tested by 7 post-menopausal women, 5 of whom were currently experiencing vulvovaginal dryness. As shown in Table 4 below, the short dome 1202 was reported to be the most comfortable contour.

**Table 4. Ranking of gel pad shapes by 7 women (1 - Favorite to 4- Least Favorite)**

| **1202** | **1204** | **1206** | **1208** |
|---|---|---|---|
| 1 | 4 | 3 | 2 |
| 4 | 2 | 3 | 1 |
| 3 | 1 | 2 | 4 |
| 1 | 4 | 2 | 3 |
| 1 | 4 | 3 | 2 |
| 2 | 1 | 3 | 4 |
| 4 | 3 | 2 | 1 |

### Ultrasound Pressure Field Mapping: Schlieren Imaging

The coupling pad allows ultrasound energy to pass through it without either (or only minimally) defocusing the beam (dispersing the energy) or inappropriately focusing the energy in an undesired location. Ultrasound pressure field maps (or Schlieren images) were recorded to examine how the prototype coupling pads affected the overall ultrasound beam profile at the desired ultrasound settings (1MHz, 1.5 W/cm² and 50% duty cycle) (see below for details on how ultrasound settings were determined).

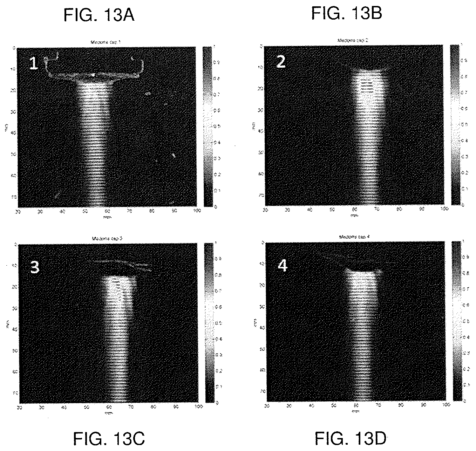

Schlieren images were recorded using an OptiSon scanner (Onda Corporation, Sunnyvale, CA). The contours from FIG. 12 were cast in 2% agarose and Shore 10A silicone and prepared for imaging. These 8 prototype coupling pads were then imaged on the OptiSon scanner. The Schlieren images shown in FIGS. 13A-13D were recorded, demonstrating the FIG. 13A depicts the Schlieren image for the short dome 1202 contour. FIG. 13B depicts the Schlieren image for the tall dome 1204 contour. FIG. 13C depicts the Schlieren image for the flat top 1206 contour. FIG. 13D depicts the Schlieren image for the oval nub 1208 contour. The images 1304, 1306 corresponding to the tall dome 1204 and the flat top 1206 shapes show the least impact on ultrasound intensity.

The images in FIGS. 13A-13D demonstrate that contour does impact ultrasound transmittance, as regions of the resulting images differ. However, these test results also reveal that the contour does not have a dramatic lensing (i.e. focusing or defocusing) effect on the beam profile. From these tests it appears that the tall dome 1204 and the flat top 1206 contours have a limited effect on the intensity, while the short dome 1202 and oval nub 1208 decreased the intensity.

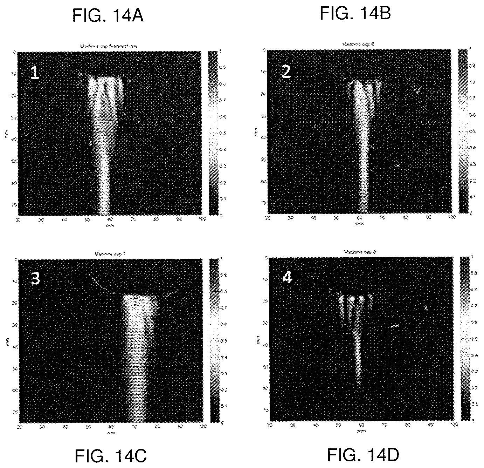

The Schlieren images shown in FIGS. 14A-14D depict the spatial intensity for each of the four coupling pad prototypes composed of Shore 10A silicone. FIG. 14A depicts the Schlieren image for the short dome 1202 contour. FIG. 14B depicts the Schlieren image for the tall dome 1204 contour. FIG. 14C depicts the Schlieren image for the flat top 1206 contour. FIG. 14D depicts the Schlieren image for the oval nub 1208 contour. These test results demonstrate that silicone dramatically attenuates the ultrasound intensity when compared to agarose.

As with FIGS. 13A-13D, it is clear from FIG. 14A-14D that the shape of the contour affects ultrasound transmittance. These images also reveal that silicone performs dramatically worse than agarose, as the intensity for each of the contours is reduced.

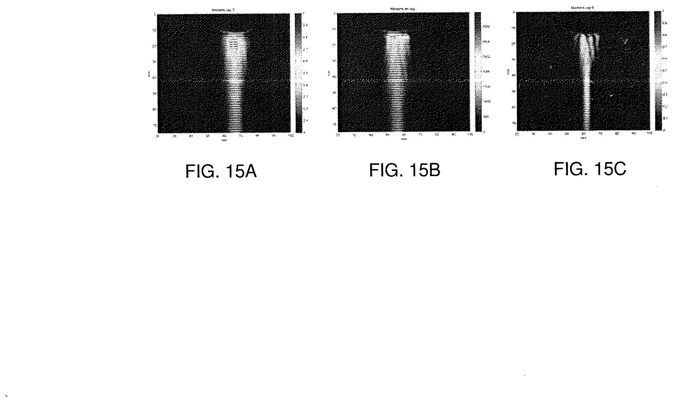

FIGS. 15A-15C further emphasize the poor performance of silicone by providing a side-by-side comparison of Schlieren images for agarose and silicone coupling Pads, as compared to the natural ultrasound beam (no coupling pad attached). FIG. 15A depicts the Schlieren image for the 2% agarose coupling pad. FIG. 15B shows the Schlieren image with no coupling pad. FIG. 15C depicts the Schlieren image for the Shore 10A silicone coupling pad. The tall dome (contour 1204 of FIG. 12) contour was used for both the agarose and silicone scans. It is clear that Silicone attenuates the ultrasound beam. Based on these test results, and the wattmeter testing discussed above, 2% agarose was identified as a preferred material for the coupling pad. However, other materials discussed herein can also be used, in some embodiments.

### Hydrophone Testing

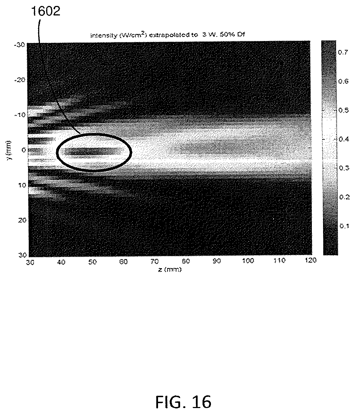

Hydrophone testing (AIMS III Hydrophone, Onda Corporation, Sunnyvale, CA) was conducted on a coupling pad design comprising 2% agarose and a flat top contour to gain a detailed understanding of the ultrasound field. The results are displayed as an intensity color map in FIG. 16. The hydrophone scanned an axial range from 30 to 120 cm from the face of the transducer and for 30 cm on each side of the ultrasound beam axis. Area 1602 represents the maximum intensity of the ultrasound's field.

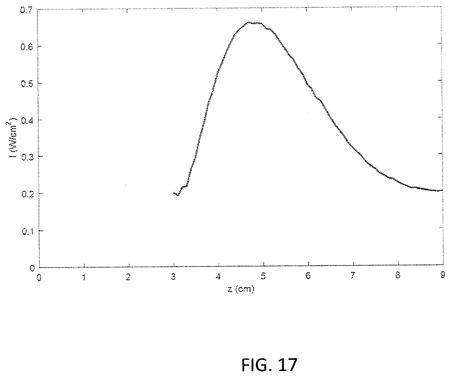

Hydrophone testing demonstrated that the ultrasound intensity is highest 40 - 60 mm from the transducer face (outlined in black), which aligns well with the vulvovaginal tissue target of the therapy. To find the exact location of maximum intensity, the hydrophone data was integrated over a 1 mm-wide cross section of the beam and plotted as a function of axial distance from the transducer face, as shown in FIG. 17. The hydrophone testing confirms that the flat top 2% agarose coupling pad deposits the maximum acoustic intensity within the target treatment window (3-5cm along the vaginal canal) at the desired ultrasound settings (1MHz, 1.5 W/cm² and 50% duty cycle). The maximum intensity occurs about 4.7 cm from the transducer face.

### Numerical Simulation of Ultrasound Device

The data collected up to this point was collected with an off-the-shelf ultrasound transducer (Intelect TranSport, Chattanooga). Thus, the data need to be scaled to fit the current ultrasound device characteristics. The only difference between the off-the-shelf system and the current ultrasound device is the transducer head size (25 vs 20 mm diameter, respectively). Therefore, numerical simulations were performed with a 20 mm ultrasound transducer to determine the location and value of maximum acoustic intensity with this transducer size.

### Validating the Numerical Simulation Code

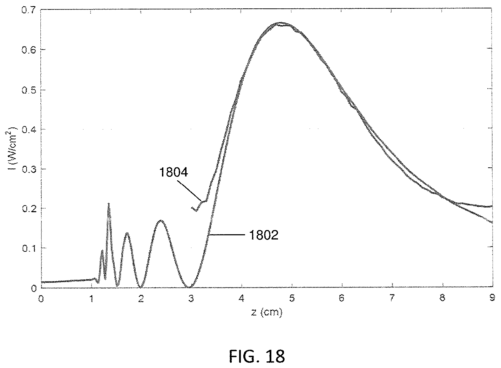

Matlab code (HIFU Simulator v1.2, Joshua Soneson, 2011) that simulates the acoustic intensity variations in different media was used for the numerical simulation. This code works by integrating the KZK-equation, which describes nonlinear wave propagation. The code was configured to run at the desired ultrasound settings (1MHz, 1.5 W/cm² and 50% duty cycle) with the incident ultrasound beam passing through two adjacent media to simulate the coupling pad and the vulvovaginal tissue. Both media were modeled as water because the body and 2% agarose are mostly water. To validate the code, coefficients were adjusted until the results with a 25 mm transducer head matched the experimental hydrophone data collected above. FIG. 18 shows a plot of the numerically simulated results (1802) along with the experimental hydrophone data (1804) as a function of axial distance from the transducer face. The experimental and simulated plots align exceptionally well in the axial range of interest (3 - 6 cm), demonstrating the robustness of the numerical model. These simulations can aid in predicting the locations of the global maximum intensities for various diameters of flat disc-type ultrasound transducers.

**Table 5: Numerical simulation constants determined for water**

| **Constant** | **Smbol** | **Value** | **Units** | **Notes** |
|---|---|---|---|---|
| Sound Speed | C | 1,480 | m/s | @ 20°C |
| Density | ρ | 1,000 | Kg/m³ | @ 20°C |
| Attenuation Coefficient | α | 0.217 | dB/m | @ 1MHz |
| Power of attenuation vs. frequency curve | η | 2.0 | - | - |
| Nonlinear parameter | β | 3.5 | - | - |

### Ultrasound Settings Considerations

### Ultrasound Settings - Literature Review and Simulations

There are four parameters that constitute the ultrasound settings. These are: treatment duration (in minutes), ultrasound frequency (in MHz), duty cycle (in % on-time) and acoustic intensity (in Watts/cm²). These settings have been informed by literature review, clinical studies, numerical simulation, and bench testing.

### Treatment Duration

Treatment duration was initially set to about 5-10 minutes or about 8 minutes, based on literature review demonstrating 5-10 minutes of ultrasound could have a profound impact on tissue blood-flow. [Baker and Bell, 1991, Dalecki, 2004] Further, clinical work has demonstrated this is a sufficient length of time to promote increases in vaginal blood-flow. This duration also meets therapy use requirements (generated through prospective user interviews) to balance benefit and total time required in order to promote user compliance.

### Ultrasound Frequency

The frequency of the ultrasound waveform delivered by the device can be about .5-2 MHz, or about 1MHz, as this frequency has been shown in the literature to penetrate tissue to a depth of 3-5 cm before attenuation.

### Duty Cycle

Duty cycle is the proportion of "on-time" of the ultrasound signal. (For example, a duty cycle of 50% means the ultrasound is pulsed and 'on' only 50% of the time.) According to the literature, pulsed ultrasound therapy (i.e. duty cycles of 20% and 50%) may have a greater effect on tissue healing than continuous wave ultrasound (duty cycle = 100%), as a duty cycle less than 100% may heighten the non-thermal biological effects.

Duty cycles of both 50% and 100% were initially tested in clinical studies. A 50% duty cycle can be preferred as numerical simulations, bench testing in tissue surrogates, and patient comments (not shown) demonstrated that duty cycles greater than 50% could lead to adverse temperature effects if the device were used incorrectly.

### Acoustic Intensity

Literature review was used to determine initial ultrasound intensities. The goal of the ultrasound therapy is to increase local vaginal blood flow 3-5 cm deep in the vaginal canal. Thus, the initial intensities to be tested were chosen based on those shown to increase blood flow at tissue depths greater than 3 cm. The acoustic intensity has been the primary parameter of interest in bench and clinical work to date.

### Bench-Testing on Tissue Surrogates: Intensity Investigation

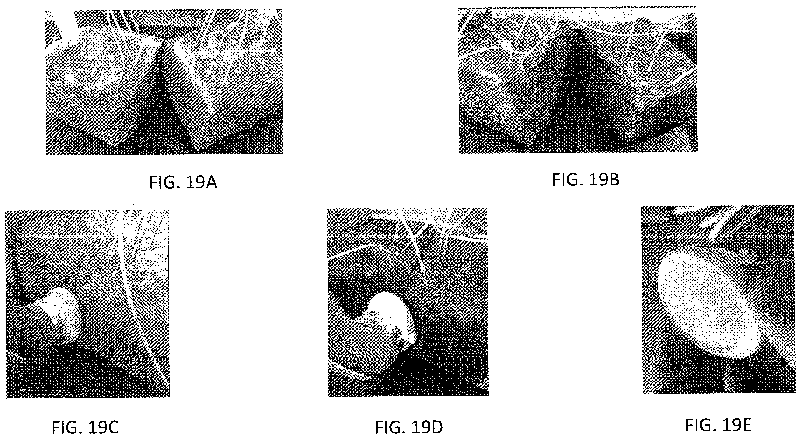

A series of bench-top experiments on tissue surrogates were run using three candidate acoustic intensities of 1.5, 2.0 and 2.2 W/cm² to observe the overall tissue temperature rise expected. The female pelvic floor tissue (including the vaginal wall tissue) is heterogeneous (predominantly composed of muscle and fat), as is the orientation of the substructure (degree of anisotropy) of each tissue component. As a rough proxy for the overall tissue response (i.e. heating), notably without the effects of blood flow that would normally be found in perfused tissue, excised pig (TS1, shown in FIGS. 19A and 19C) and cow (TS2, shown in FIGS. 19B and 19D) tissue with a medium-light level of adipose content were used as tissue surrogates (TS). The tissue was gradually brought to 37°C (body temperature) in a water bath just prior to ultrasound testing.

The tissue was cut to an appropriate size to represent the vaginal opening (shown in FIGS. 19A and B), with two larger pieces held adjacent to each other, as shown in FIGS. 19C and D. The Flat Top coupling pad (shown in FIG. 19E) made from 2% agarose was used with the Intelect TranSport^{®} (Chattanooga) ultrasound machine, and set to a frequency of 1 MHz, 50% duty cycle, and a treatment duration of 8 minutes. The coupling pad was then placed at the split line between the two pieces and held with a small pressure so as to maintain acoustic coupling to the tissue (i.e. no air gaps), as shown in FIGS. 19C and 19D. A grid of thermocouples (TC) was placed at 1.5 cm increments and 2 cm depths along the length of the simulated vaginal canal, on either side of the split line. Temperature profiles were recorded for both TS1 and TS2 at the three candidate acoustic energies.

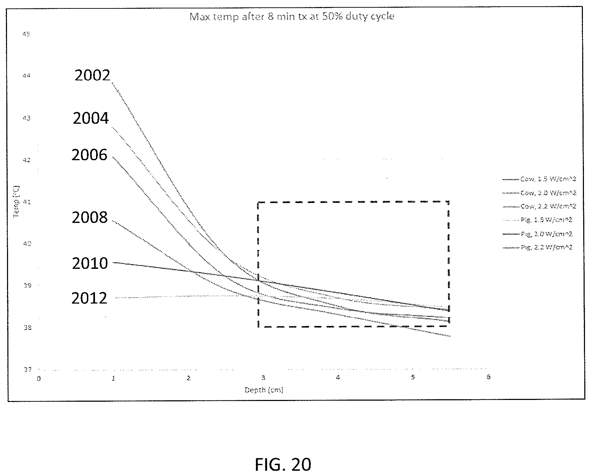

The results of these tests are depicted in FIG. 20. As shown, the temperature change at the target therapeutic depths of 3 to 6 cm was largely independent of the set acoustic intensity for each tissue. However, the temperature within the first few centimeters was significantly influenced by the ultrasound intensity. Note that the temperature of living (perfused) tissue is predicted to be about one degree Celsius cooler than that of non-perfused tissue, due to the cooling effects of blood-flow. Hence, these results indicated the ultrasound intensity should be limited to less than 2.0 W/cm², in some embodiments.

### Transducer Size, Shape, and Array

As with the ultrasound settings, the transducer configuration for the ultrasound therapy has been influenced by previous and ongoing clinical studies. Efficacy of the chosen transducer to create the desired therapeutic effect was paramount, but business considerations regarding the price of different types of transducers (e.g. curved versus flat) were also considered.

### Transducer Shape

The shape of the ultrasound transducer dictates the form of the emitted ultrasound beam, and thus both curved and flat transducers were considered. Curved transducers are designed to precisely focus an ultrasound beam at a therapy target. Though this transducer shape showed initial promise, it may be impractical, in some embodiments, for a home-use therapy. It was determined that, in some embodiments, the curved transducer could easily deposit too much energy in one location of the vaginal canal if used outside of a well-controlled environment. Furthermore, the clinical work has demonstrated that a diffuse application of ultrasound can, in some embodiments, provide better therapy by vasodilating as much of the vascular bed of the vaginal canal as possible.

Flat, disc-type transducers were considered and proved appropriate for the therapy application, as they can achieve the desired therapy effect and are cost-effective. Flat transducers are characterized by a 'natural focal length,' which is a function of the transducer size and ultrasound frequency. At this focal length acoustic intensity reaches a global maximum as the ultrasound beam transitions from a near-field signal (characterized by intensity turbulence) to a far-field, smooth and predictable signal. As the therapy can be a fixed frequency (e.g., 1 MHz) in some embodiments, the natural focal length for the transducer can be tuned by adjusting the transducer size. Thus, a flat, disc-type ultrasound transducer can be used.

### Transducer Size

As described, a flat disc-type transducer's region of maximum intensity is driven by its size, and thus the next step is to determine the diameter that can yield both the desired natural focal length of 3 to 6 cm (our target zone) and can be driven at a resonant 1 MHz frequency. The clinical studies to date have largely used a 25 mm flat disc-type diameter transducer with an effective radiating area (ERA) of 5 cm². Based on literature review and user interview research, it has been determined that this size may be slightly too large, in some embodiments. Therefore, a slightly smaller transducer (diameter 20 mm, with an ERA of 3.14 cm²) was investigated.

The computational model validated above (HIFU Simulator v1.2, Joshua Soneson, 2011) was used to model and compare the following three transducer diameters:
- Target size based on user research = 20 mm diameter (ERA = 3.14 cm²)
- Clinical study size = 25 mm diameter (ERA = 5 cm²)
- Larger size for bench marking = 35 mm diameter (ERA = 10 cm²)

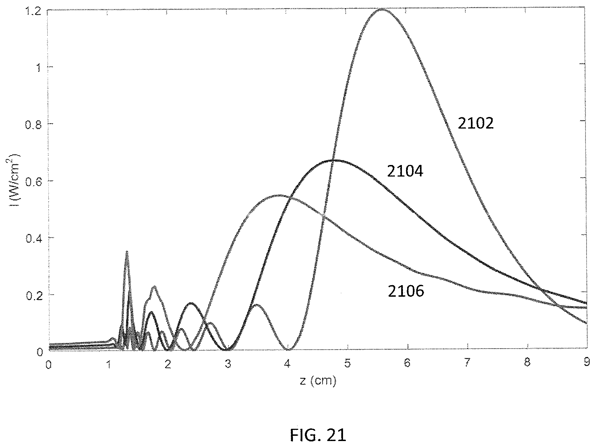

The results are presented in FIG. 21, and demonstrate that the maximum acoustic intensity for the transducer with ERA = 3.14 cm² occurs between 3 and 6 cm, which is exactly our target location in the vaginal canal. Both of the other transducers modeled have maximums that may be too deep for the desired therapy effect, in some embodiments.

### Transducer Array

In order to achieve adequate acoustic effect in our target axial distance of 3 to 5cm, hydrophone experiments showed that a simple flat, disc-type transducer worked sufficiently well. Although these flat transducers are not intended for focusing the ultrasound energy, they do have a 'natural focal length' where the cleanest waveform and maximum intensity are achieved. The flat disc-type transducers were appropriate for producing a more diffuse spread of energy in the near-field axial region (from the transducer face). Further, from a business model perspective, these single disc transducers are more cost effective to manufacture and implement (i.e. their driving electronics are simpler) than any array could be.

### Development of a Coupling Pad Mold

Clear propagation of ultrasonic energy from the surface of the transducer through the coupling pad medium and into the target tissue can be dependent on the ultrasound waves encountering a minimized number of air gaps, bubbles, or defects, along the direction of travel. Each air gap or defect can cause incident energy to attenuate from scattering or absorption; thereby weakening the ultimate dose to the intended area.

### Ported Cover

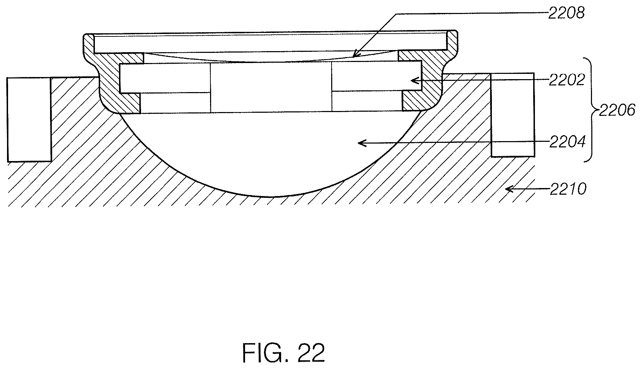

The coupling pad can be molded onto a support ring configured to provide structure to the coupling pad component. The support ring and coupling pad can together form the disposable component of the handheld device. Early efforts at molding the coupling pad into the support ring produced a significant meniscus in the coupling pad, which resulted in an air gap at the interface of the assembled transducer and coupling pad. Liquid coupling pad material can be cured in a mold (e.g., 3D printed mold). FIG. 22 shows the problematic meniscus 2208 formed in the curing coupling pad 2206, formed by the support ring 2202 and the liquid coupling material 2204 sitting in the mold 2210. In addition, curing of the coupling pad material 2204 in these molds 2210 frequently lead to trapped air bubbles within the coupling pad.

In order to minimize these deleterious effects, the following three molds were designed and fabricated.

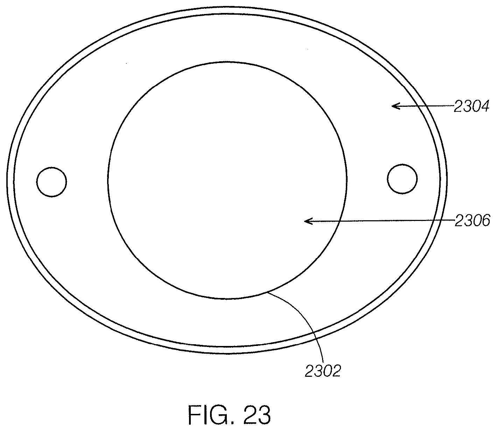

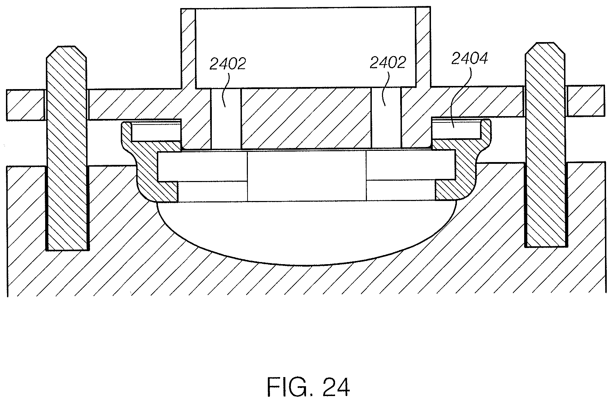

To prevent the wall surface tension from forming a problem meniscus in an open-cavity mold, a top plate was added to the mold assembly. As shown in FIG. 23, this plate covers the hole 2302 in the support ring 2304 forming the transducer interface 2306 where the ultrasound transducer will subsequently seat against the coupling pad. FIG. 24 illustrates an embodiment of this mold cover design. Two ports holes 2402 are added to the plate 2404, so air and excess materials can flow out of the mold freely. Once hardened, the two port plugs, now full of material, can be sheared off, revealing a flat, even surface.

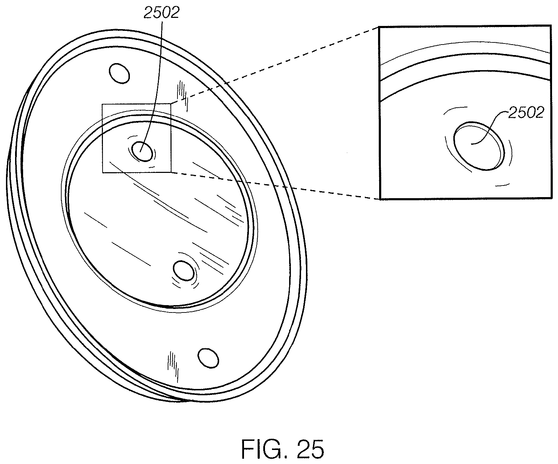

Although the Ported Cover design effectively eliminated the problem meniscus, the two ports can leave small blemishes on the surface of the cured agarose that contacts the transducer face, as shown in FIG. 25. These "rough spots" 2502 are undesirable, as they can cause a non-uniform seating of the transducer face to the agarose coupling pad and hence, a possible reduction in the ultrasound transmission through the coupling pad.

### Ported Support Ring

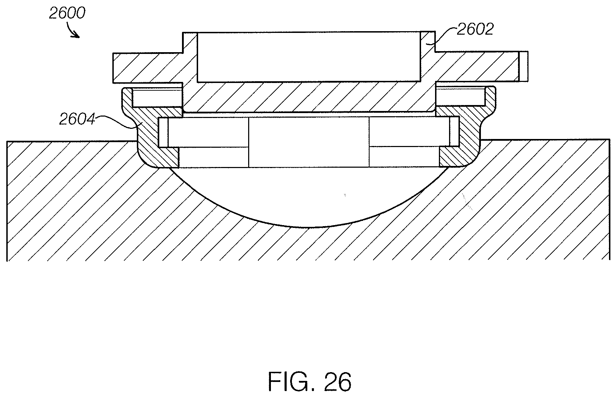

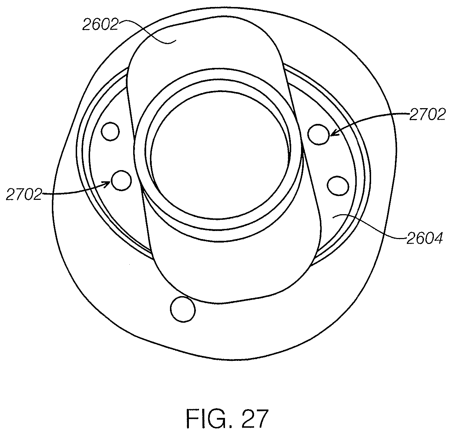

In the embodiment shown in FIGS. 26 and 27, the mold 2600 uses the same top plate 2602 that is used in mold 2400 of FIG. 24 in order to prevent the meniscus. The key difference in mold 2600 is that the port holes 2702 are now placed into the support ring 2604, itself, shown in FIG. 27. This change of port placement minimizes the problems that were encountered in mold 2400, and ensures a smooth transducer coupling surface.

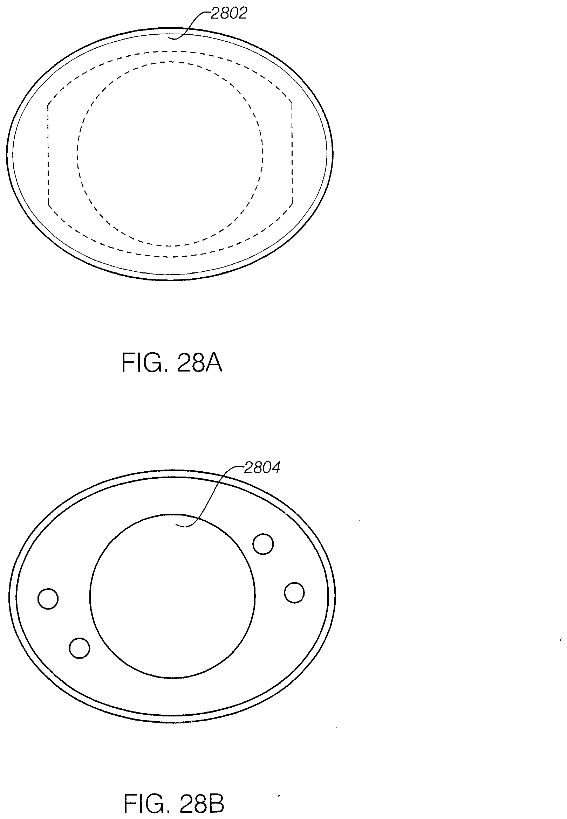

In mold 2600, the liquid coupling medium flows in through one port in the support ring 2604, fills the mold 2600, and flows out through the second vent hole when the mold cavity is full. This flow helps prevent air bubbles from being trapped in the cured coupling pad. This design consistently created bubble-free coupling pads more often than any previous mold design. A finished coupling pad/support ring assembly 2802 made using mold 2400 shown in FIGS. 28A and 28B. Note the planar, smooth rear surface 2804 of the coupling pad where the transducer face seats on assembly.

### Top Fill

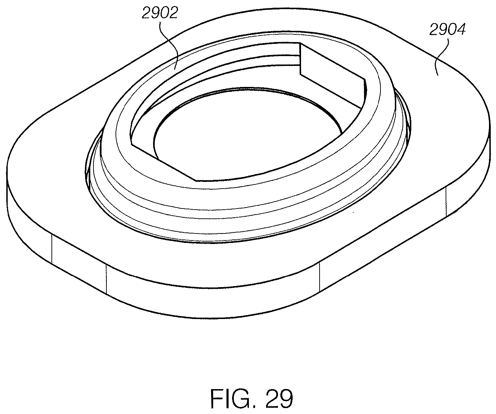

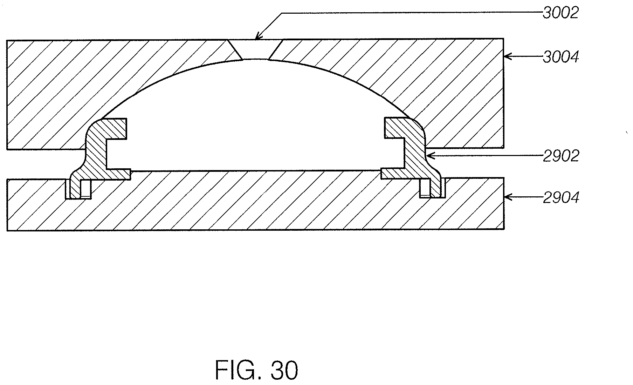

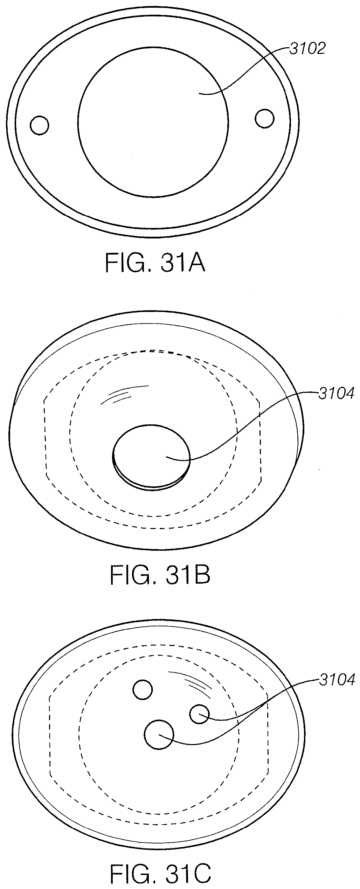

The top fill mold 3000 design, shown in FIGS. 29 and 30, takes the original mold design and flips the fill location 180 degrees. In this design, the ring 2902 snaps into a bottom plate 2904 or mold base, shown in FIG. 29. The liquid coupling medium 3004 flows in through a hole 3002 in the mold top 3003 leading to the top surface of the coupling pad and support ring assembly (FIG. 30). As shown in FIG. 31A, the top fill mold 3000 design can produce a perfectly flat and smooth surface 3102 that interfaces with the transducer. A potential downside to this design is that voids 3104 can form during curing, as shown in FIGS. 31B and 31C.

### Component Packaging

In some embodiments, the component packaging comprises separate packaging for each disposable portion (e.g., support ring and coupling pad). The individualized packaging can hold the disposable portion in a liquid medium to keep the coupling pad hydrated as it may dry out if exposed to air. In some embodiments, the hydrating solution comprises a bacteriostatic solution (e.g., 0.9% benzyl alcohol solution). The support ring comprises plastic, in some embodiments, and will not degrade in the hydrating solution. The support ring can also provide a strong, rigid support for the coupling pad.

In some embodiments, the component packaging can comprise a blister pack formed to the shape of the coupling pad component. The shelf life can be about 1 - 3 years (e.g., 1 year, 2 years, 3 years). The blister pack can provide an easy to open component packaging sealed to lock in a hydrating solution. The blister pack can be rigid enough to support the shape of the coupling pad. The blister pack can comprise a medical grade plastic with a backing of foil or foil lined paper. A flexible backing can help with ease of opening for the user while still trapping the coupling pad moisture in the package.

In some embodiments, the coupling pad must be disinfected, but does not require sterilization

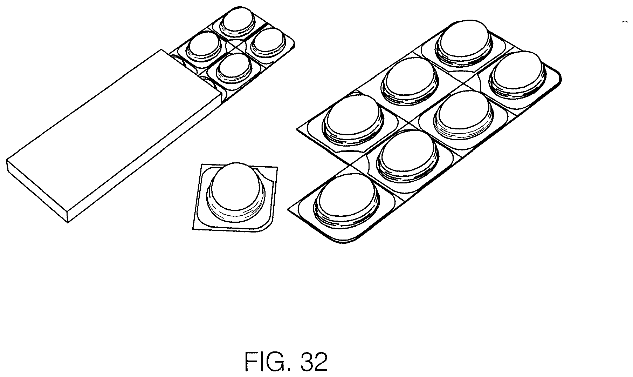

An embodiment of component packaging is provided by the Stephen Gould Corporation. The packaging consists of a thermoformed tray that holds 8 gel pads shown in FIG. 32, a box, and the easy to remove backing.

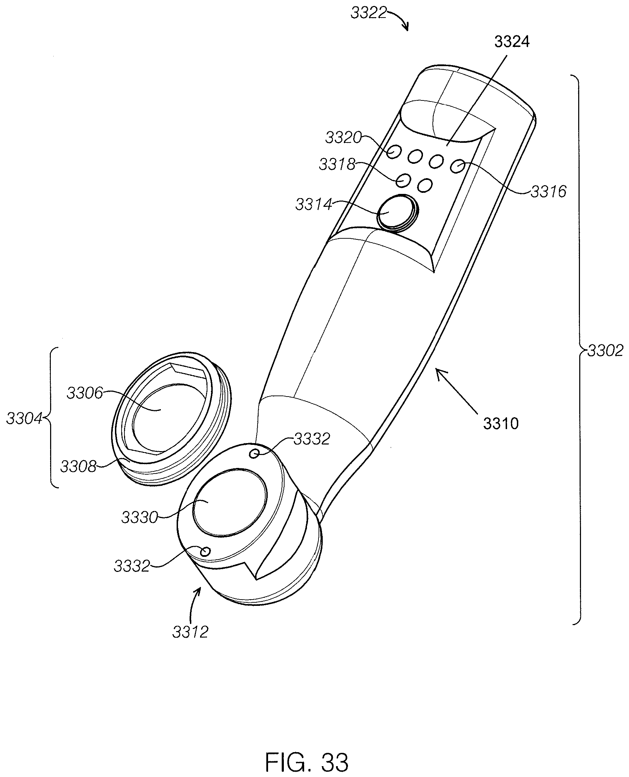

FIG. 33 illustrates another embodiment of an ultrasound therapy device 3300. The device 3300 comprises a main device portion 3302 and a coupling pad component 3304. Unless described otherwise, the device 3300 can comprise features or combinations of features of other ultrasound therapy devices described herein.

The coupling pad component 3304 comprises a coupling pad 3306 and a support ring 3308. As described herein, the coupling pad 3306 can be formed from a coupling pad material (e.g., liquid medium). The support ring 3308 can provide structure to the coupling pad and aid in its formation. In some embodiments, the coupling pad component is disposable.

The main device portion 3302 comprises a handle portion 3310 and a head portion 3312. The handle portion 3310 can be configured to fit in a patient's hand. The handle portion 3310 may comprise one or more controls or buttons.

### Additional Embodiments

Additional embodiments of ultrasound devices are provided below. It will be appreciated that the various embodiments devices can comprise features or combinations of features described herein with respect to other embodiments of devices.

As shown in FIG. 33, the handle portion 3310 comprises a single button 3314. In some embodiments, the device 3300 can be activated by pressing button 3314. The device can be turned on and off by pressing button 3314. Turning on the device can initiate energy delivery at pre-set conditions and for a pre-set duration. In some embodiments, the device can be paused by pressing button 3314. For example, holding down button 3314 for a period of time (e.g., 1 second, 2 seconds, 3 seconds) can cause the device to be turned on and/or off. Simply pressing the button 3314 can pause ongoing energy delivery. Pressing the button 3314 again can resume treatment at the point at which it was paused.

The handle portion 3310 can comprise one or more indicator lights. As shown in FIG. 33, the handle portion 3310 can comprise a power indicator light 3316 configured to be illuminated when the device is on. The handle portion 3310 can comprise a battery indicator light 3318 configured to indicate battery status. The battery indicator light 3318 can change color to indicate battery status. For example, green can indicate a full charge. Yellow can indicate the device needs to be charged soon. Red can indicate the device does not have sufficient power to complete a therapy session. The handle portion 3310 can also comprise one or more 'therapy remaining" lights 3320 to indicate time left in treatment. The lights 3320 can all be illuminated at the start of treatment, and can shut off, one at a time, to indicate the amount of time that has passed. For example, given an 8 minute treatment time, all four lights 3320 are illuminated at the start of treatment. Every 2 minutes, one of the lights 3320 shuts off until they are all off at the end of the treatment. In some embodiments, the handle portion 3310 comprises 1, 2, 3, 4, 5, or 6 therapy remaining indicator lights.

As noted above, the handle portion 3302 can be configured to be held in a patient's hand during treatment. As such, the handle portion 3302 would be held near the front of the groin area. The controls and/or indicator lights can be positioned towards an end 3322 of the handle portion away from the head portion in order to provide a better view and easier access to the patient.

The control and/or indicator lights can be positioned in a recessed area 3324, as shown in FIG. 33. Such positioning can help a user easily maneuver their fingers to the area. A single button control can also allow the user to manage functionality of the device without needing a clear view of the controls.

In some embodiments, the controls or indicators can comprise a user interface for user input of treatment parameters. In some embodiments, the device can be remotely controlled by a smartphone, dedicated device controller, computer, tablet, or the like. In some embodiments, the device comprises digital displays indicating battery status, therapy remaining status, or device status.

In some embodiments, the head portion 3312 of the device 3300 comprises an ultrasound head 3330, as shown in FIG. 33. The ultrasound head 3330 can comprise an ultrasound transducer. The ultrasound transducer can be a flat, disc-type transducer. Other configurations (e.g., curved) are also possible. In some embodiments, the ultrasound transducer is a ceramic piezoelectric crystal. Other transducers are also possible. The transducer can have a diameter of about 15-25 mm, about 15 mm, about 20 mm, or about 25 mm. Other transducer sizes are also contemplated. The effective radiating area (ERA) of the transducer can be about 2-12 cm², 2-8 cm², 2-6 cm², 2-4 cm², or about 3 cm². In some embodiments, the ERA is about 3.14 cm². The beam non-uniformity ratio (BNR) of the transducer can be about 6:1. In some embodiments, the natural focal length is about 30-100 mm, about 40-90 mm, about 50-80 mm, about 55-75 mm, about 60-70 mm, or about 65 mm.

The head portion 3312 also comprises attachment means for connecting to the coupling pad portion 3304. As shown in FIG. 33, the attachment means can comprise magnets on the head portion 3312 configured to engage with magnets 3332 (not shown) on the coupling pad portion 3304. Magnet attachment means can be easy to use and easy to clean as they may have a low profile. Other attachment means (e.g., hook and loop, snaps, straps, etc.) are also possible. For example, in some embodiments, the attachment means comprises a threaded connection between the head portion 3312 and the coupling pad portion 3304. The threaded connection can require a small turn to engage the two components. For example, ¼ turn or ½ turn can be used to engage the two components and put the coupling pad 3306 in acoustic connection with the ultrasound transducer 3330.

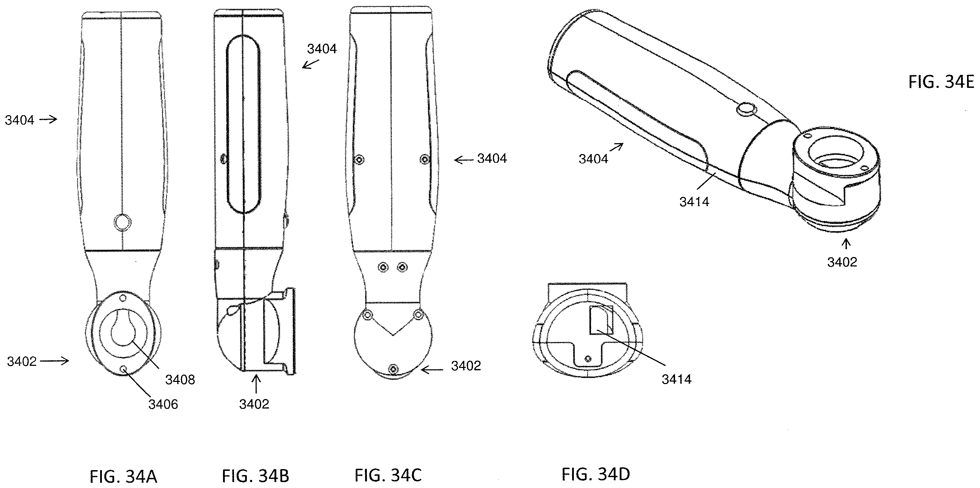

FIGS. 34A-E illustrate various views of an embodiment of a main device portion 3400 without the coupling pad portion 3304. FIG. 34A is a top view of the main device portion 3400 comprising a head portion 3402 and a handle portion 3404. The head portion 3402 comprises attachment means (e.g., magnets) 3406 for attaching head portion 3402 and coupling pad portion. The head portion 3402 also comprises an ultrasound head comprising an ultrasound transducer face 3408 for contacting a coupling pad. An outer portion of the head portion 2402 around the transducer face 3408 contacts the support ring when the coupling pad portion is attached to the head portion 3402.

FIG. 34B is a side view of the main device portion. The main device 3400 can comprise plastic (e.g., Polyethylene, Polypropylene, Polystyrene, Polyester, Polycarbonate, Polyvinyl Chloride, Polymethylmethacrylate (PMMA), Polyetheretherketone (PEEK), etc.) with a silicone overmold 3410, shown in FIG. 34B, over certain portions of the handle portion 3404 for ease and comfort during use. FIG. 34C shows a back view of the main device 3400, showing the head portion 3402, handle portion 3404 and silicone overmold 3410. The device can have a length of about 150-250 mm, 175-225 mm, about 185 mm, 190 mm, 195 mm, 200 mm, 205, mm, or 210 mm. The device can have a width of about 20-60mm, 40-50mm, or about 46mm. The device can have a thickness of about 20-60mm, 40-50mm or about 43mm.

As shown in FIG. 34C, the base of the head portion 3402 comprises a greater surface area or diameter than a top part of the head portion, near the transducer face 3408. This greater area at the base of the head portion 3402 can allow for ultrasound transducer components and circuitry while still allowing a more compact shape for the portion of the head potion 3402 that will be near the treatment area. It will be appreciated that, in some embodiments, ultrasound circuitry is provided external to the device.

FIG. 34D depicts an end or back view of the main device 3400, looking towards the handle portion 3404. The bottom surface 3414 of the device 3400 can comprise a port 3412 that can be used for charging the device. The port 3412 can be used to charge a rechargeable battery in the device via a corded connection. A standard outlet or USB can be used the charge the device. In some embodiments, the device can be used when plugged in. In some embodiments, the device cannot be used when plugged in.

FIG. 34E shows a top perspective view of device 3404 showing handle portion 3404 and head portion 3402. Control or button 3406 is shown on handle portion 3402. Control 3406 is shown closer to head portion 3402 than in the embodiment of FIG. 33. Handle portion can also comprise indicator lights, which are not shown in FIG. 34E. Handle portion 3404 extends towards the head portion and tapers at a neck portion 3414 of the device 3400. The smaller diameter of the neck potion 3414 can allow for ease of positioning the head portion at the treatment area. In some embodiments, the neck portion or another portion of the device comprises a strain gauge to inform the user if the head portion is being placed into sufficient contact with the treatment area.

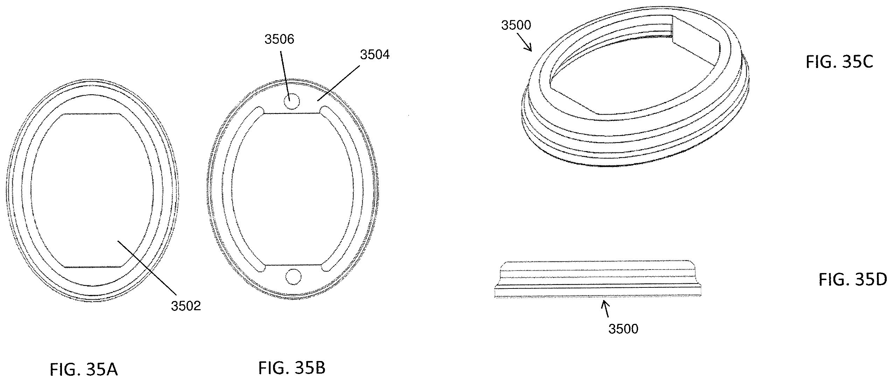

FIGS. 35A-35D show various views of an embodiment of a support ring 3500 before coupling pad material has been molded into the support ring. FIG. 35A shows a top view of the support ring 3500. The ring 3500 comprises an ovular shape in FIG. 35A, but other shapes (e.g., circular, rectangular, square, etc.) are also contemplated. A length of the support ring 3500 can be about 30-60 mm, about 40-50 mm, about 45 mm, about 46 mm, or about 47 mm. In some embodiments, the length of the support ring 3500 is about 46.6 mm. A width of the support ring 3500 can be about 20-50 mm, about 30-40 mm, about 35 mm, about 36 mm, or about 37 mm. In some embodiments, the width of the support ring 3500 is about 36.48 mm. The opening 3502 in support ring 3500 will be filled in by coupling pad material and, thus, represents the area of the coupling pad that will engage the transducer face. Other support ring designs may comprise plates or other features obscuring a portion of window 3502. By not having such a feature, better coupling between the transducer and coupling pad is enabled. Additionally, not having such a feature improves manufacturability as injection molding can be used and material cost decreases. In some embodiments, not having a backing plate or other similar feature in the support ring can cause the coupling pad to fall out of the support ring if handled too often or too rigorously. In single use embodiments, this can be a nice feature to prevent re-use of the coupling pad. The support ring can comprise a plastic material (e.g., HDPE, Polyethylene, Polypropylene, Polystyrene, Polyester, Polycarbonate, Polyvinyl Chloride, Polymethylmethacrylate (PMMA), Polyetheretherketone (PEEK), etc.).

FIG. 35B shows a bottom view of the support ring 3500. The bottom surface 3504 can be configured to mate with the head portion of the main device. The bottom surface 3504 comprises attachment means 3506, such as magnets, configured to engage attachment means of the head portion of the main device. As noted above, other attachment means, such as a threaded connection, are also possible.

FIG. 35C depicts a top perspective view of the support ring 3500. The support ring 3500 can have rounded edges along a perimeter of the device to improve user comfort. FIG. 35D depicts a side view of the device. FIG. 35D shows a slight taper to the shape of the device as it extends from the bottom surface towards the top surface. This shape can allow for good connection to the head portion while maintaining comfort for the user.

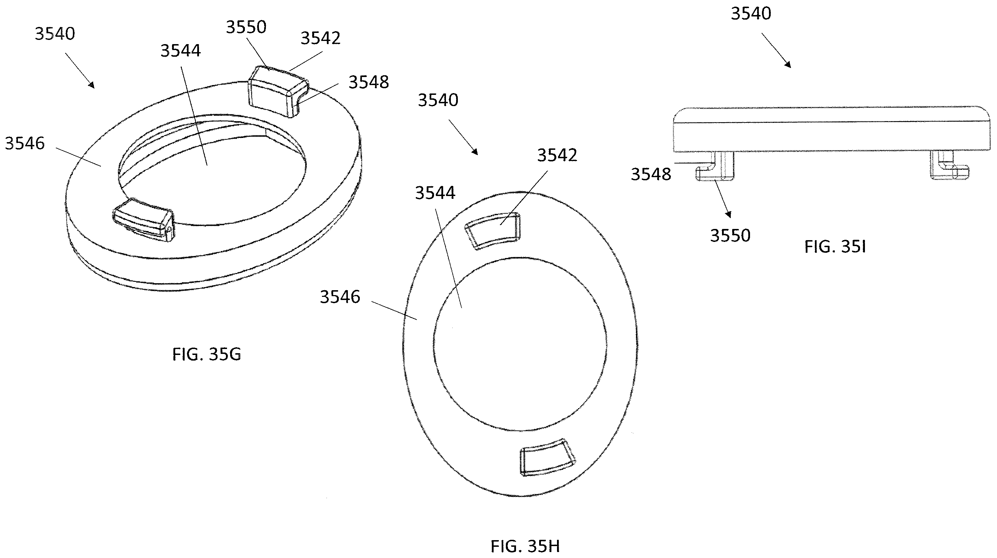

FIGS. 35E-35I illustrate another embodiment of an attachment means for attaching a support ring and coupling pad to an ultrasound transducer head. FIGS. 35E and 35F depicts various views of an ultrasound device transducer support 3530. FIG. 35E shows a top perspective view of the transducer support 3530; and FIG. 35F shows a top view of the transducer support 3530. The device transducer support 3530 is the portion of the ultrasound device that will connect to the coupling pad component creating the acoustic coupling between the transducer and the coupling pad. The transducer support 3530 includes an opening 3532 in the center, in which the transducer will be positioned. Thus, the dimensions of the opening 3532 can correspond to the dimensions of the transducer face. The inner surface of the opening 3532 can also be configured to correspond to the shape of the transducer. The transducer support 3530 extends from the opening 3532 in a radial direction from the center of the opening to create a ring around the opening 3532. This surface 3533 can comprise an ovular shape as shown in FIGS. 35E and 35F. Other shapes (e.g., rectangular, square, circular, etc.) are also possible. The transducer support 3530 comprises two slots 3534 comprising a wide portion 3536 and a narrow portion 3538. The slots 3534 are configured to engage tabs 3542 on the support ring embodiment shown in FIGS. 35G-35I.

FIGS. 35G-35I illustrate various views of an embodiment of a support ring 3540. FIG. 35G shows a top perspective view of the support ring; FIG. 35H shows a top view of the support ring; and FIG. 35I shows a side view of the support ring. The support ring 3540 comprises an opening 3544 configured to receive a coupling pad. This area represents the area of the coupling pad that will interface with the transducer. The support ring 3540 extends outwardly from the opening 3544 in a radial direction from the center of the opening 3544. This surface 3546 of the support ring 3540 will interface with the transducer support, for example shown in FIGS. 35E and 35F. The support ring 3540 comprises an ovular shape in FIGS. 35G-35I, but other shapes are also possible, as described above. In some embodiments, the shape of the support ring and the transducer face are configured to correspond such that one doesn't extend much past the other. This correspondence can create a more comfortable experience for the user as the device will have a sleek exterior. The support ring 3540 comprises tabs 3542 configure to interact with slots 3534 on the transducer face. A narrow portion 3548 of the tab extends from support ring surface 3546 and widens to form a wide portion 3550 of the tab. The wide portion 3550 of the tab can be configured to be inserted into the wide portion 3536 of a slot on the transducer support. Rotating the support ring 3540 relative to the transducer support (e.g., ¼ turn, ½ turn, ¾ turn, 1 turn) can slide narrow portion 3548 of the tab into the narrow portion 3538 of the slot, locking the support ring and transducer support in place relative to one another. The tabs 3542 are shown with a shape resembling a portion of an annulus. Other shapes are also possible.

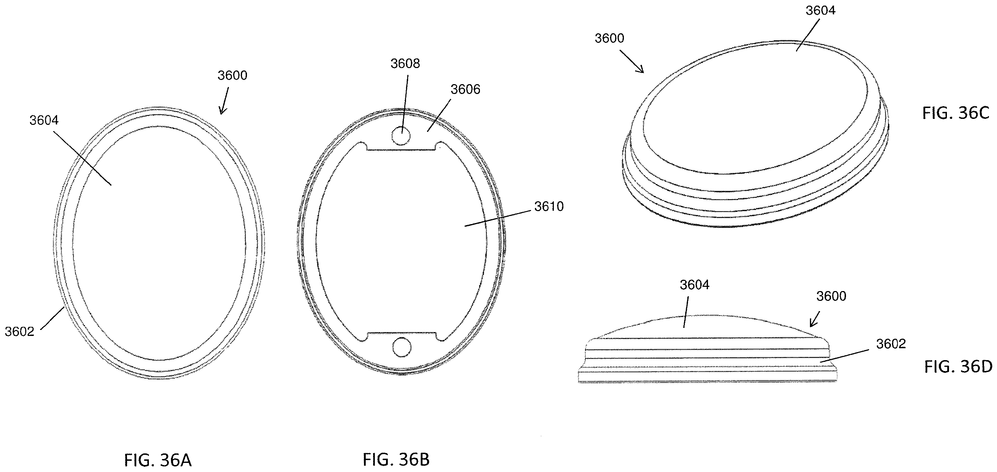

FIGS. 36A-D depict various views of a coupling pad portion comprising a support ring 3602 with a coupling pad 3604 molded into the support ring. FIG. 36A illustrates a top view of the coupling pad portion 3600 comprising the coupling pad 3604 and the support ring 3602. The coupling pad can have a length of about 30-50 mm, about 35-55 mm, or about 40 mm. The coupling pad can have a width of about 20-40 mm, about 25-35 mm, or about 30 mm. The top surface of the coupling pad shown in FIG. 36A is the portion configured to engage the treatment area of the patient. The coupling pad can be molded from a liquid coupling material which can comprise one or a combination of agarose (e.g., 2-3% agarose, 1-2% agarose, 3-4% agarose or 2.5% agarose), silicone (e.g., with a Shore 00-20, Shore 10A, Shore 20A, or Shore 30A hardness), and water. Other materials are also possible, as described above. In some embodiments, the coupling pad comprises 2% agarose with a lower concentration agarose coating the top and bottom surface to increase their lubricity.

FIG. 36B illustrates a bottom view of the coupling pad portion 3600. FIG. 36B shows a bottom surface 3606 of the support ring. The bottom surface 3606 comprises attachment means, such as magnets 3608, configured to engage with corresponding attachment means on a head portion of the main device. The bottom surface 3610 of the coupling pad represents that area that will interface with the ultrasound transducer. In some embodiments, a surface area of the coupling pad that interfaces with the ultrasound transducer is about 3-7 cm², 4-6 cm², 5 cm². In some embodiments, the surface area is about 4.9 cm².

FIG. 36C shows a top perspective view of the coupling pad portion 3600 comprising coupling pad 3604 and support ring 3602. FIG. 36D depicts a side view of the coupling pad portion 3600 comprising coupling pad 3605 and the support ring 3602. The coupling pad can have a convex, rounded, dome shape. The coupling pad can have a height (above support ring) of about 1-7 mm, about 2-6 mm, about 3-5 mm, or about 4 mm. As described elsewhere herein, this configuration of the coupling pad can be most comfortable and intuitive to use for patients. The shape of the coupling pad can allow the user to self-navigate the device to the proper position near the vagina, at the introitus. In some embodiments, the user navigates to the proper positioning based solely on touch; thus, intuitive positioning can be useful for ensuring proper device use. Other coupling pad configurations are also possible (e.g., taller or shorter dome, central nub, flat top dome, ridge, etc.)

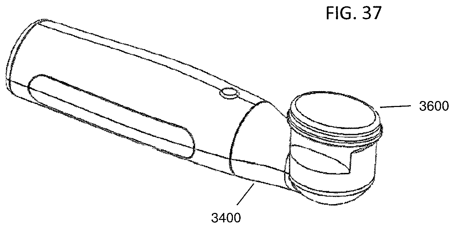

FIG. 37 illustrates a top perspective view of an assembled ultrasound device comprising coupling pad portion 3600 attached to main device portion 3400.

The following figures depict further embodiments of a coupling pad. It can be important for the coupling pad to maintain self-lubrication throughout the duration of the treatment. Maintained lubrication can prevent hot spots caused by ultrasound standing waves and can promote better acoustic coupling to the patient's tissue.

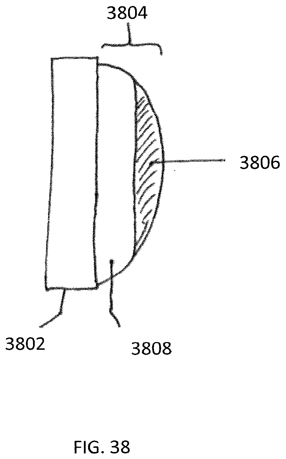

FIG. 38A shows an embodiment of a coupling pad component 3800 comprising a support ring 3802 and a coupling pad 3804. The coupling pad 3804 comprises an outer portion 3806 and an inner portion 3808. The outer portion 3806 is configured to interface with the patient's tissue. The inner portion 3808 is positioned closer to the support ring and the device. The outer portion 3806 can comprise a different material from that of the inner portion 3808. In some embodiments, the outer portion 3806 is configured to change phase from solid to liquid at or around (e.g., just above) body temperature. For example, the material can be solid phase at around 70°-85°F. The material can melt at about 85°F. This phase change to liquid can provide a layer of lubrication between the inner portion 3808 and the patient's tissue. In some embodiments, the colors of the two materials are different so a user can quickly see that a pad has been used. For example, in some embodiments, the inner portion 3808 can comprise agarose and the outer portion 3806 can comprise coconut oil. Generally, coconut fats belong to the unique group of vegetable oils called lauric oil about 44 - 51 %. Lauric acid (CH3(CH2)10COOH) is known as small molecule fatty acid (< 14:0) which contains short or medium chain of saturated fatty acid. Other chemical compositions of coconut oil belong to myristic acid (16 - 19 %), caprylic acid (9.0 - 9.5 %), palmitic acid (8.0 - 9.5 %), oleic acid (5 - 6 %), capric acid (5 - 10 %), steric acid (3.0 - 3.5 %) and linoleic acid (1.0 - 1.5 %), respectively. Other materials for the inner portion 3808 are also possible, such as those described above with respect to coupling pad materials.

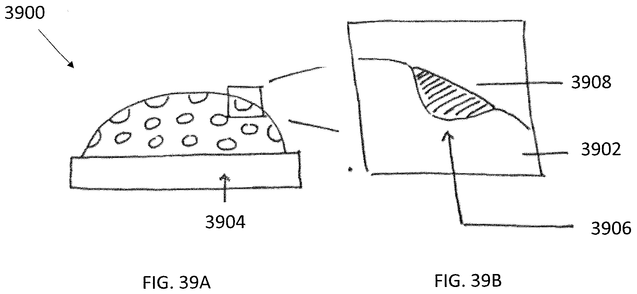

FIGS. 39A and 39B illustrate another embodiment of a coupling pad component 3900 comprising a coupling pad 3902 and a support ring 3904. The coupling pad 3904 comprises pockets 3906 pre-formed in the coupling pad. FIG. 39B shows an expanded view of pocket 3906. The pockets 3906 can be filled with an ultrasound conductive medium 3908 (e.g., ultrasound gel, mineral oil, or other sonolucent and viscous material). Including the ulstrasound conductive material in pre formed pockets can prevent lubrication from an ultrasound conductive material (e.g., ultrasound gel) from rubbing off at first contact with the patient's tissue, which can thereby enhance acoustic coupling during treatment. The pockets can be shaped as circles, spheres, ellipsoids, ellipses, or have other configurations. The pockets can be about 0.5-3 mm in diameter.

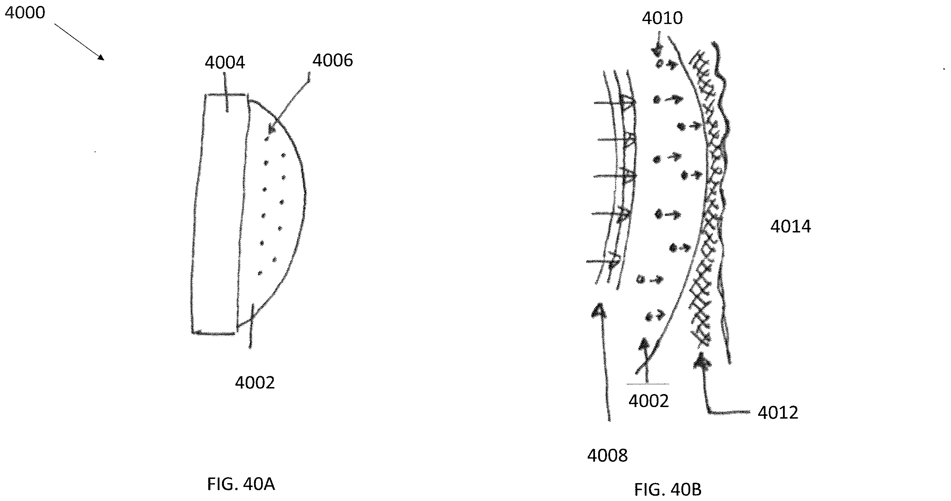

FIGS. 40A and 40B show another embodiment of a coupling pad component 4000 comprising a coupling pad 4002 and a support ring 4004. The coupling pad 4000 comprises an additive 4006 (e.g., a lubricant). The additive can be driven to an outer surface of the coupling pad when the ultrasound is active via the mechanism of sonophoresis. This action is shown in FIG. 40B. An ultrasound wave 4008 is shown propagating towards the outer surface of the coupling pad. Arrows 4010 indicate the movement of the additive causing a layer of lubrication 4012 at the patient's tissue. The sonophoresis of the additive can allow the coupling pad to maintain a layer of lubrication between the outer surface of the coupling pad and the patient's tissue over the course of the treatment, which can maintain and/or enhance acoustic coupling.

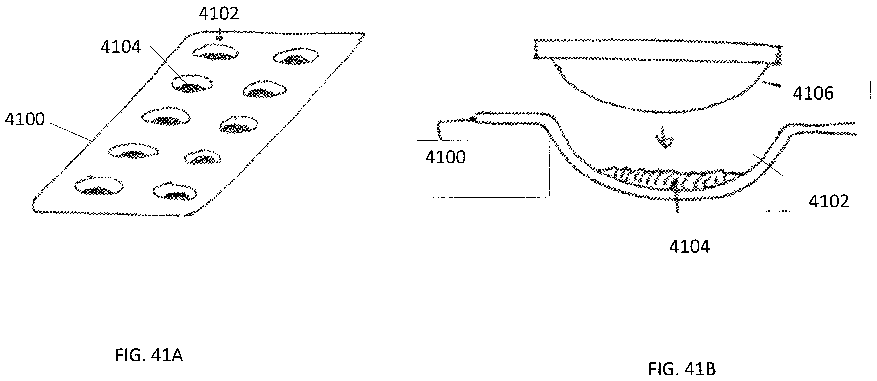

FIGS. 41A and 41B show an embodiment of coupling pad component packaging that can aid in lubrication of the coupling pad. As shown in FIG. 41A, a blister pack tray 4100 for holding the coupling pad component is provided. Each depression 4102 in the tray if filled with an ultrasound conductive material 4104 (e.g., ultrasound gel, mineral oil, etc.). FIG. 41B shows how the coupling pad component is placed in the blister pack tray 4100. The outer surface of the coupling pad 4106 is placed into the ultrasound conductive material 4104 in the depression 4102. This type of storage can 'prime' the coupling pad by adding a thin film of lubricant to the outer surface, which can enhance acoustic coupling between the coupling pad component and the patient's tissue.

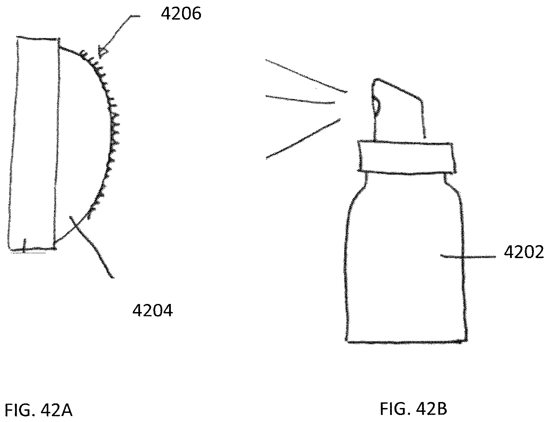

FIGS. 42A and 42B illustrate an embodiment in which a spray bottle of lubricant 4202, shown in FIG. 42B, is used to spray lubricant onto a surface of the coupling pad 4204. Adding lubricant can add a layer of lubricant 4206 to an outer surface of the coupling pad, shown in FIG. 42A. Other means for adding a layer of lubricant are also possible (e.g., squirt bottle, pre-lubricated wipes or pads, etc.).

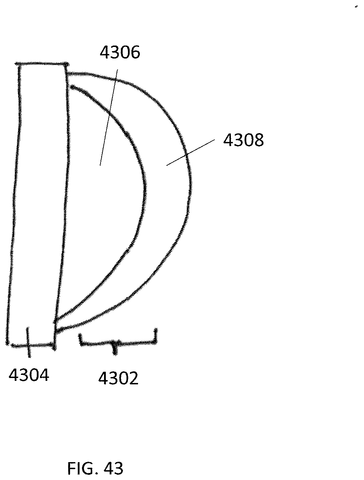

FIG. 43 illustrates an embodiment of a coupling pad component 4300 comprising a support ring 4304 and a coupling pad 4302. In this embodiment, the coupling pad comprises two different materials, as described above. A first portion 4306 of the coupling pad comprises a first material, and a second portion 4308 of the coupling pad comprises a second material, different from the first. The different materials can comprise different concentrations of the same material. For example, the first portion 4306 can comprise 2% agarose; and the second portion can comprise 0.5% agarose. The second portion 4308, which contacts the patient's tissue, can comprise a more lubricious material than the first portion 4306, which can provide enhanced acoustic coupling.

### Alternative Coupling Pad Component Embodiments

The following figures depict an additional coupling pad component embodiments. The coupling pad components shown below can allow for attachment to any ultrasound transducer head. Unless otherwise described, the coupling pad component can comprise one or a combination of features of other coupling pads and related coupling pad components described herein.

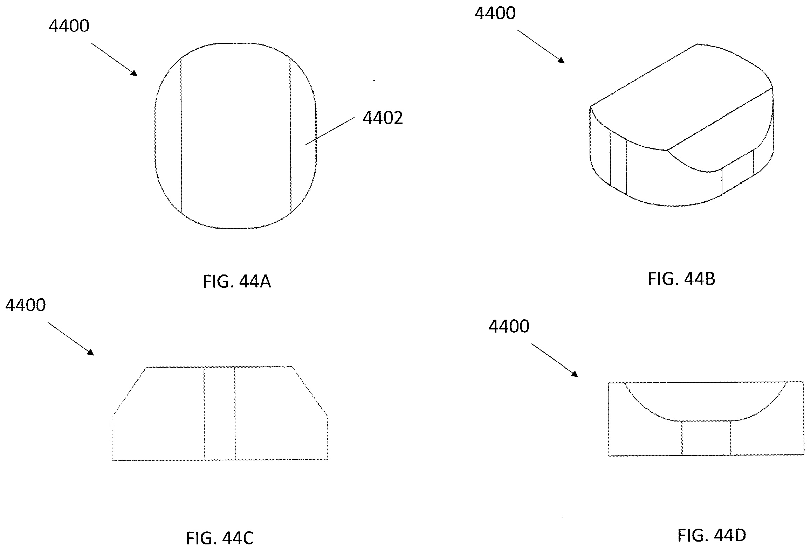

FIGS. 44A-44D illustrate various views of a coupling pad 4400. FIG. 44A shows a top view of the coupling pad 4400. The coupling pad 4400 comprises a generally rectangular shape with rounded corners. This shape can provide a large surface area to interface with patient tissue and with an ultrasound transducer while still maintaining a smooth exterior. Other shapes (e.g., ovular, square, rectangular) are also possible. The coupling pad 4400 can comprise chamfered edges 4402 which can help secure the device in a coupling pad holder. FIG. 44B shows a top perspective view of the coupling pad. The top surface of the coupling pad is the portion configured to interface with the patient's tissue. The coupling pad can have a thickness of about 5-25 mm, about 10-20 mm, or about 15mm. The coupling pad can have a length of about 25-55 mm, about 30-50 mm, about 35-45 mm, or about 40mm. The coupling pad can have a width of about 15-45 mm, about 20-40 mm, about 25-35 mm, or about 30mm. A length less than about 40mm can help prevent ultrasound application to the urethra or clitoris and can help with self-navigation. FIG. 44C shows a front view of the coupling pad, showing the chamfered edge 4402. The edge 4402 can be chamfered at an angle of about 35-55 degrees or about 45 degrees. FIG. 44D shows a side view of the coupling pad 4400.

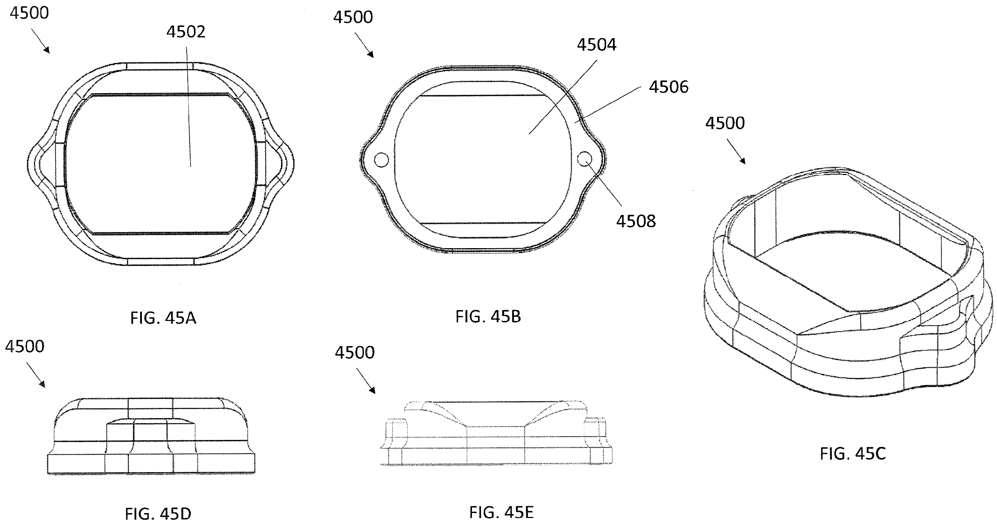

FIGS. 45A-45D illustrate various views of an embodiment of a top portion 4500 of a coupling pad holder. FIG. 45A shows a top view of the top portion 4500. The top surface includes opening 4502 through which the top surface of the coupling pad is to be inserted. The opening 4502 is about 25-55 mm, about 30-50 mm, about 35-45 mm, or about 40mm long. The opening 3502 is about 15-45 mm, about 20-40 mm, about 25-35 mm, or about 30 mm wide. FIG. 45B shows a bottom view of the top portion 4500. The bottom surface comprises opening 4504 within which a bottom surface of the coupling pad will sit. In some embodiments, the bottom surface 4506 of the coupling pad is configured to be flush with the bottom surface 4506 of the top portion 4500. The opening 4504 represents the portion of the coupling pad that will interface with the ultrasound transducer. The bottom surface 4506 can comprise an attachment mechanism, such as magnets 4508, configured to interact with a corresponding mechanism on a bottom portion of the coupling pad holder. The bottom view of FIG. 45B also shows that the bottom opening 4504 is greater than the top opening 4502. This smaller top opening allows the chamfered edges of the coupling pad to seat in the top opening 4502. FIG. 45C shows a top perspective view of the top portion 4500. The top portion 4500 can generally have rounded surfaces and edges, providing comfort to the user during use of the device. FIG. 45D illustrates a front view of the device; and FIG. 45E illustrates a side view of the device, also showing the rounded surfaces and edges.

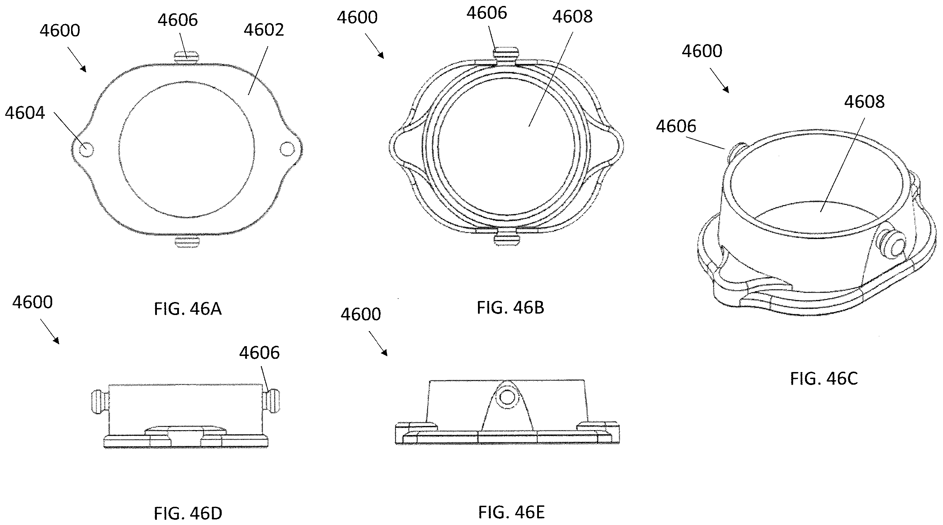

FIGS. 46A-46E show various views of an embodiment of a bottom portion 4600 of a coupling pad holder. FIG. 46A shows a top view of the bottom portion 4600. A top surface 4602 of the bottom portion 4600 is configured to interact with a bottom surface 4506 of the top portion 4500. An attachment mechanism, such as magnets 4604 are configured to interact with a corresponding attachment mechanism on the bottom surface of the top portion of the holder. Other attachment mechanisms, such as side tabs or those described elsewhere herein, are also possible. Protrusions or knobs 4606 are provided on either side of the bottom portion 4600. These knobs 4606 can be configured to hold or attach to a strap that is used to secure the coupling pad holder to an ultrasound transducer device. FIG. 46B shows a bottom view of the bottom portion 4600. Opening 4608 is configured to receive an ultrasound transducer portion (e.g., head) of an ultrasound device (e.g., ultrasound wand). FIG. 46C shows a bottom perspective view of the bottom portion 4600. The opening 4608 is shown as round, but other configurations (ovular, square, rectangular) are also possible. FIGS. 46D and 46E show front and side views, respectively, of the bottom portion.

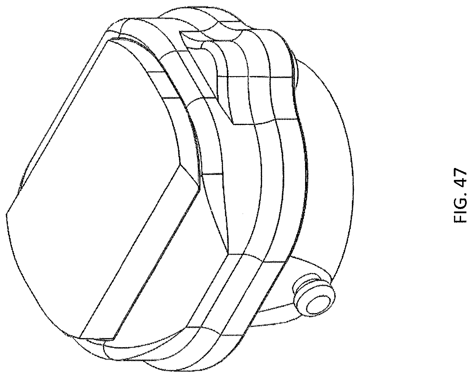

FIG. 47 depicts top perspective view of an embodiment of a coupling pad component 4700 comprising a coupling pad 4400 positioned within assembled coupling pad holder 4702 comprising top and bottom portions. A top surface of the coupling pad, including a portion of chamfered edges 4402 is shown protruding through the opening 4502 in the top portion of the coupling pad holder. As described above, the coupling pad component comprises rounded edges and surfaces providing comfort to the user during use.

To assemble the coupling pad component, a user can place a top surface of a coupling pad through a top opening of a top portion of the coupling pad holder. The bottom surface of the coupling pad should be flush with the bottom edge of the top portion of the holder. This positioning can ensure good contact with the ultrasound transducer. The user can then connect the bottom portion of the holder to the top portion using an attachment mechanism, such as magnets shown in FIGS. 45B and 46A. In some embodiments, the user can then place an ultrasound conductive material, such as ultrasound gel through the bottom opening of the bottom holder onto the bottom surface of the coupling pad. The user can then insert an ultrasound transducer portion of an ultrasound device through the bottom opening of the bottom holder so that it contacts a bottom surface of the coupling pad. A strap attached to knobs on the bottom portion of the holder can be used to secure the coupling pad component to the ultrasound device.

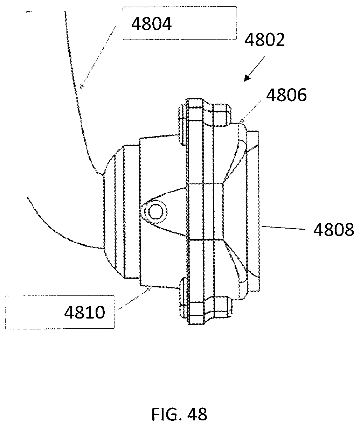

FIG. 48 illustrates an embodiment of an assembled coupling pad component 4802, including top portion 4806 and bottom portion 4810 of the holder and coupling pad 4808, attached to an ultrasound transducer device 4804.

A method of using a device as described herein follows. A user ensures the device is sufficiently charged to initiate a therapy session. The user can remove a coupling pad portion from its packaging and attach it to the head portion of the device. Attaching can be performed using magnets, a threaded connection, or as otherwise described herein. Once the device is assembled, the user holds the handle portion and positions the device so the coupling pad is in contact with her introitus (vaginal opening). The user then activates the device. Activating the device can comprise pressing down a button on the handle portion. In some embodiments, the button is held down to turn the device on or off. For example, the button can be depressed for about 1, 2, 3, or more seconds. During treatment pressing the same button can pause and resume treatment. Pressing the button can activate the device for the desired duration and at the desired settings.

In some embodiments, the ultrasound settings comprise a frequency of about 1 MHz. The intensity can be about 1.5 W/cm². The duty cycle can be about 50%. In some embodiments, the frequency can be .5 MHz-3 MHz, 1.5 MHz, 2 MHz, or 2.5 MHz. In some embodiments, the intensity can be bout 1-2.5 W/cm², 1 W/cm², 1.5 W/cm², 2 W/cm², 2.2 W/cm², or 2.5 W/cm². In some embodiments, the duty cycle can be between about 20%-80%, about 30%, about 40%, about 60%, about 70%, about 80%, about 90%, or about 100%.

In some embodiments, the device is used daily for eight minutes per day. As described herein, in other embodiments, the device can be used multiple times a day, weekly, bi-weekly, monthly, etc. The device can be used for different durations. For example, durations of 5, 6, 7, 9, 10, or 10-15 minutes are contemplated.

Two acute and one chronic (ongoing) IRB-approved clinical studies have been conducted at Stanford University Hospital. The goal of the first study (Acute Study #1) was to determine therapy safety, as therapeutic ultrasound had never been used in this part of the body for this purpose with this patient population.

Safety was demonstrated by Acute Study #1. The results showed that the energy used may be too low and therefore attenuated before reaching the target depth of 3 cm to 6 cm. Hence, a second acute clinical study (Acute Study #2) was conducted at increased ultrasound intensities (See Table 6), still deemed to be safe based on numerical and benchtop temperature simulations (not shown). The data from this study showed a significant (3x) increase in vaginal tissue blood flow and temperature (about 2.5°) (data not shown), demonstrating the current device mechanism of action. Results from Acute Study #1 are partly shown in FIG. 9.

**Table 6: Summary of Ultrasound Settings in the clinical studies**

| **Trial** | **No. pts treated** | **Frequency** | **Intensity** | **Duty Cycle** | **Duration** |
|---|---|---|---|---|---|
| Acute Study #1 | 10 | 1 MHz | 1.5 W/cm² | 50% | 8 min. |
| Acute Study #2 | 9 | 1 MHz | 2.2 W/cm² | 100% | 8 min. |
| Chronic Study | 7 | 1 MHz | 1.5 W/cm² | 50% | 8 min., daily |
| | 8 to 20 | 1 MHz | 2.0 W/cm² | 50% | 8 min., daily |

Patient symptoms, as recorded by surveys, also showed improvements in both Acute Study #1 and #2. 68% of participants reported an increased level of vulvovaginal lubrication after treatment for 24 hours or more after the study visit.

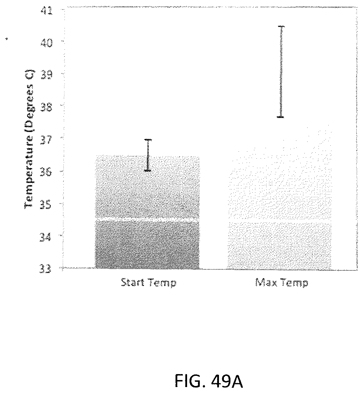

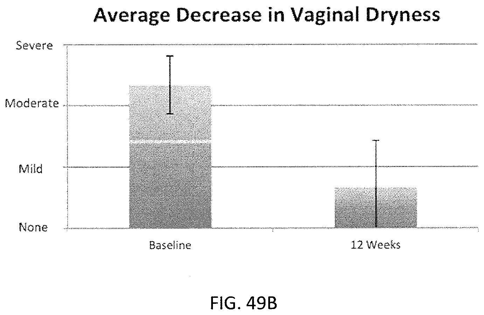

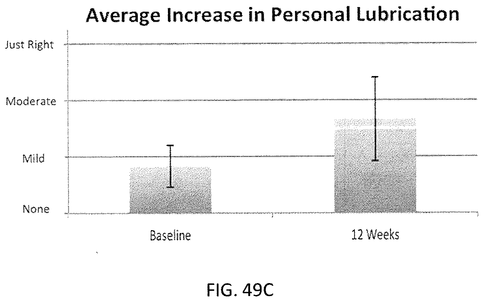

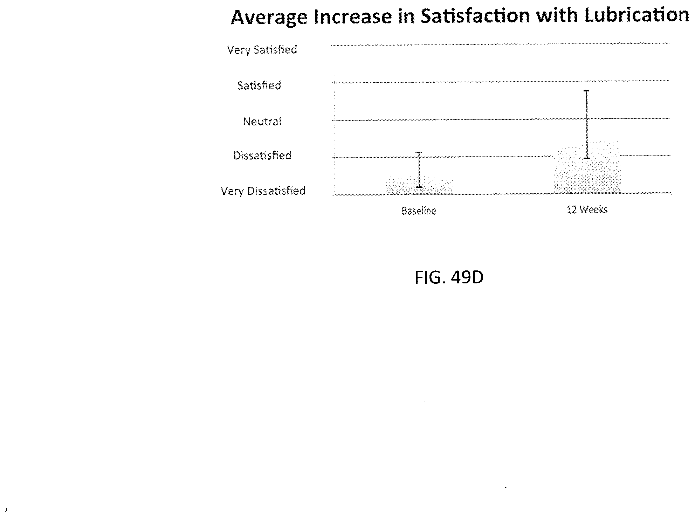

To determine if repeated use of the current ultrasound therapy will lead to improvements in VVA, a third clinical study (Chronic Study, Table 6) is currently being conducted. In this investigation, participants use a ultrasound treatment prototype at home, daily for 8 minutes a day. For this study, the Ultrasound Settings were modified slightly from Acute Study #2. Duty cycle was reduced from 100% to 50%. Intensity was decreased to 1.5 W/cm² for the first seven patients. After it was clear this energy level was well tolerated (no complaints or adverse events), the dose was escalated to 2.0 W/cm² for all subsequent pts. FIGS. 49A-49D show results from this study. The error bars in the tables represent the standard deviation. FIG. 49A shows the mean change in temperature after 8 minutes of ultrasound therapy. FIG. 49B depicts an average decrease in vaginal dryness for patients who have completed the study to date. As shown, the patients' generally report vaginal dryness has clearly decreased over 12 weeks. FIG. 49C shows an average increase in personal lubrication for patients who have completed the study to date. As shown, patients generally report an increase in personal lubrication after 12 weeks. FIG. 49D depicts an average increase in satisfaction with lubrication for patients who have completed the study to date. As shown, patients are generally more satisfied with their lubrication after 12 weeks of therapy.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected," "attached" or "coupled" to another feature or element, it can be directly connected, attached or coupled to the other feature or element or intervening features or elements may be present. In contrast, when a feature or element is referred to as being "directly connected," "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a," "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under," "below," "lower," "over," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly," "downwardly," "vertical," "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims that follow, unless the context requires otherwise, the word "comprise," and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/-10% of the stated value (or range of values), etc. Any numerical range recited herein is intended to include all sub-ranges subsumed therein.

Although various illustrative embodiments are described above, any of a number of changes may be made to various embodiments. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes**.**

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure.

## Claims

1. A handheld device (100) for vulvovaginal rejuvenation, comprising:
an ultrasound energy source (102);
an energy delivery element (112) comprising a conformable coupling pad (114) sized and shaped to engage tissue in or around the subject's vagina and external genitalia, the energy delivery element further comprising a support ring supporting the coupling pad and comprising an attachment means to detachably connect the coupling pad to the ultrasound energy source with the coupling pad in contact with a face of the ultrasound energy source, the coupling pad comprising a dome shaped contour and adapted to enhance coupling of the energy delivery element with the tissue in or around the subject's vagina and external genitalia; and
a handle (120) configured for maintaining the position of the device during use;
wherein the ultrasound energy source (102) is configured to deliver ultrasound energy through the energy delivery element to the tissue in or around the subject's vagina and external genitalia to rejuvenate said tissue.

2. The device of claim 1, further comprising a coupling assembly (3332) configured to couple the energy delivery element to the handheld device.

3. The device of claim 2, wherein the coupling assembly comprises magnets (3332) arranged and configured for magnetically coupling to corresponding magnets on a portion of the energy delivery element.

4. The device of claim 1, wherein the coupling pad comprises a coupling medium.

5. The device of claim 4, wherein the coupling medium comprises a hydrogel.

6. The device of claim 1, wherein the coupling pad has a thickness of 3-5 mm.

7. The device of claim 1, wherein the coupling pad has a length of about 35-45 mm.

8. The device of claim 1, wherein the coupling pad has a width of about 25-35 mm.

9. The device of claim 1, wherein the energy source (102) comprises an ultrasound generator adapted to provide ultrasound energy at a frequency of about .5 MHz to 2 MHz.

10. The device of claim 1, wherein the energy source (102) comprises an ultrasound generator configured to provide ultrasound energy at a duty cycle of about 20-80%.

11. The device of claim 1, wherein the energy source (102) comprises an ultrasound generator configured to provide ultrasound energy at a period of 2 to 10 minutes, every day, multiple times a day, every few days, once a week, once every couple of weeks, or once a month.

12. The device of claim 1, wherein the energy source (102) comprises an ultrasound generator configured to provide ultrasound energy at an intensity of about 1.0-2.2 W/cm².

13. The device of claim 1, wherein the energy source (102) has an effective radiating area (ERA) of about 2-8 cm².

14. The device of claim 1, further comprising a feedback mechanism configured for alerting the subject of insufficient contact between the energy delivery element (112) and the tissue in or around the subject's vagina and external genitalia.

15. The device of claim 1, further comprising a sensor configured to measure a physiological parameter of tissue in or around the subject's vagina and external genitalia relating to vaginal rejuvenation when the energy delivery element is engaged with tissue in or around the subject's vagina and external genitalia, the device being further configured to use information from the sensor to control energy delivery from the energy delivery element.

## Patentansprüche

1. Handheld-Vorrichtung (100) zur vulvovaginalen Verjüngung, umfassend:
eine Ultraschallenergiequelle (102);
ein Energieabgabeelement (112), das ein anpassungsfähiges Kopplungskissen (114) umfasst, das so bemessen und geformt ist, dass es mit dem Gewebe in oder um die Vagina und die äußeren Genitalien der Person in Eingriff kommt, wobei das Energieabgabeelement ferner einen Stützring umfasst, der das Kopplungskissen stützt, und ein Befestigungsmittel umfasst, um das Kopplungskissen lösbar mit der Ultraschallenergiequelle zu verbinden, wobei das Kopplungskissen in Kontakt mit einer Fläche der Ultraschallenergiequelle steht, wobei das Kopplungskissen eine kuppelförmige Kontur aufweist und geeignet ist, die Kopplung des Energieabgabeelements mit dem Gewebe in oder um die Vagina und die äußeren Genitalien der Person zu verbessern; und
einen Griff (120), der so konfiguriert ist, dass er die Position der Vorrichtung während der Verwendung beibehält;
wobei die Ultraschallenergiequelle (102) so konfiguriert ist, dass sie Ultraschallenergie durch das Energieabgabeelement an das Gewebe in oder um die Vagina und die äußeren Genitalien der Person abgibt, um das Gewebe zu verjüngen.

2. Vorrichtung nach Anspruch 1, die ferner eine Kopplungsanordnung (3332) umfasst, die so konfiguriert ist, dass sie das Energieabgabeelement mit der Handheld-Vorrichtung koppelt.

3. Vorrichtung nach Anspruch 2, wobei die Kopplungsanordnung Magnete (3332) umfasst, die so angeordnet und konfiguriert sind, dass sie mit entsprechenden Magneten an einem Teil des Energieabgabeelements magnetisch gekoppelt werden.

4. Vorrichtung nach Anspruch 1, wobei das Kopplungskissen ein Kopplungsmedium umfasst.

5. Vorrichtung nach Anspruch 4, wobei das Kopplungsmedium ein Hydrogel umfasst.

6. Vorrichtung nach Anspruch 1, wobei das Kopplungskissen eine Dicke von 3-5 mm aufweist.

7. Vorrichtung nach Anspruch 1, wobei das Kopplungskissen eine Länge von etwa 35-45 mm aufweist.

8. Vorrichtung nach Anspruch 1, wobei das Kopplungskissen eine Breite von etwa 25-35 mm aufweist.

9. Vorrichtung nach Anspruch 1, wobei die Energiequelle (102) einen Ultraschallgenerator umfasst, der geeignet ist, Ultraschallenergie mit einer Frequenz von etwa 0,5 MHz bis 2 MHz bereitzustellen.

10. Vorrichtung nach Anspruch 1, wobei die Energiequelle (102) einen Ultraschallgenerator umfasst, der so konfiguriert ist, dass er Ultraschallenergie mit einem Tastverhältnis von etwa 20-80 % bereitstellt.

11. Vorrichtung nach Anspruch 1, wobei die Energiequelle (102) einen Ultraschallgenerator umfasst, der so konfiguriert ist, dass er Ultraschallenergie in einem Zeitraum von 2 bis 10 Minuten, jeden Tag, mehrmals am Tag, alle paar Tage, einmal pro Woche, einmal alle paar Wochen oder einmal im Monat bereitstellt.

12. Vorrichtung nach Anspruch 1, wobei die Energiequelle (102) einen Ultraschallgenerator umfasst, der so konfiguriert ist, dass er Ultraschallenergie mit einer Intensität von etwa 1,0 bis 2,2 W/cm² bereitstellt.

13. Vorrichtung nach Anspruch 1, wobei die Energiequelle (102) eine effektive Strahlungsfläche (ERA) von etwa 2-8 cm² aufweist.

14. Vorrichtung nach Anspruch 1, die ferner einen Rückkopplungsmechanismus umfasst, der so konfiguriert ist, dass er die Person auf einen unzureichenden Kontakt zwischen dem Energieabgabeelement (112) und dem Gewebe in oder um die Vagina und die äußeren Genitalien der Person aufmerksam macht.

15. Vorrichtung nach Anspruch 1, die ferner einen Sensor umfasst, der so konfiguriert ist, dass er einen physiologischen Parameter des Gewebes in oder um die Vagina und die äußeren Genitalien der Person in Bezug auf die vaginale Verjüngung misst, wenn das Energieabgabeelement mit dem Gewebe in oder um die Vagina und die äußeren Genitalien der Person in Kontakt ist, wobei die Vorrichtung ferner so konfiguriert ist, dass sie Informationen von dem Sensor verwendet, um die Energieabgabe von dem Energieabgabeelement zu steuern.

## Revendications

1. Dispositif portatif (100) destiné au rajeunissement vulvo-vaginal, comprenant :
une source d'énergie ultrasonore (102),
un élément de transmission d'énergie (112) comprenant un coussinet de couplage conformable (114) dont les dimensions et la forme lui permettent de venir au contact des tissus situés dans ou autour du vagin et des organes génitaux externes du sujet, l'élément de transmission d'énergie comprenant en outre un anneau de support soutenant le coussinet de couplage et comprenant un moyen de fixation pour relier de manière amovible le coussinet de couplage à la source d'énergie ultrasonore, le coussinet de couplage étant en contact avec une face de la source d'énergie ultrasonore, le coussinet de couplage comprenant un contour en forme de dôme et étant adapté à améliorer le couplage de l'élément de transmission d'énergie avec les tissus situés dans ou autour du vagin et des organes génitaux externes du sujet, et
une poignée (120) conçue pour maintenir le dispositif en position pendant son utilisation ;
la source d'énergie ultrasonore (102) étant conçue pour fournir de l'énergie ultrasonore à travers l'élément de transmission d'énergie aux tissus situés dans ou autour du vagin et des organes génitaux externes du sujet afin de rajeunir lesdits tissus.

2. Dispositif selon la revendication 1, comprenant en outre un ensemble de couplage (3332) conçu pour coupler l'élément de transmission d'énergie au dispositif portatif.

3. Dispositif selon la revendication 2, dans lequel l'ensemble de couplage comprend des aimants (3332) agencés et conçus pour se coupler magnétiquement à des aimants correspondants sur une portion de l'élément de transmission d'énergie.

4. Dispositif selon la revendication 1, dans lequel le coussinet de couplage comprend une substance de couplage.

5. Dispositif selon la revendication 4, dans lequel la substance de couplage comprend un hydrogel.

6. Dispositif selon la revendication 1, dans lequel le coussinet de couplage présente une épaisseur de 3 à 5 mm.

7. Dispositif selon la revendication 1, dans lequel le coussinet de couplage présente une longueur d'environ 35 à 45 mm.

8. Dispositif selon la revendication 1, dans lequel le coussinet de couplage présente une largeur d'environ 25 à 35 mm.

9. Dispositif selon la revendication 1, dans lequel la source d'énergie (102) comprend un générateur d'ultrasons adapté à fournir une énergie ultrasonore à une fréquence d'environ 0,5 MHz à 2 MHz.

10. Dispositif selon la revendication 1, dans lequel la source d'énergie (102) comprend un générateur d'ultrasons conçu pour fournir une énergie ultrasonore selon un cycle de service d'environ 20 à 80 %.

11. Dispositif selon la revendication 1, dans lequel la source d'énergie (102) comprend un générateur d'ultrasons conçu pour fournir de l'énergie ultrasonore sur une période de 2 à 10 minutes, tous les jours, plusieurs fois par jour, tous les quelques jours, une fois par semaine, une fois toutes les deux semaines ou une fois par mois.

12. Dispositif selon la revendication 1, dans lequel la source d'énergie (102) comprend un générateur d'ultrasons conçu pour fournir une énergie ultrasonore à une intensité d'environ 1,0 à 2,2 W/cm².

13. Dispositif selon la revendication 1, dans lequel la source d'énergie (102) présente une surface de rayonnement effective (ERA, effective radiating area) d'environ 2 à 8 cm².

14. Dispositif selon la revendication 1, comprenant en outre un mécanisme de retour d'information conçu pour alerter le sujet en cas de contact insuffisant entre l'élément de transmission d'énergie (112) et les tissus situés dans ou autour du vagin et des organes génitaux externes du sujet.

15. Dispositif selon la revendication 1, comprenant en outre un capteur conçu pour mesurer un paramètre physiologique des tissus situés dans ou autour du vagin et des organes génitaux externes du sujet, concernant le rajeunissement vaginal lorsque l'élément de transmission d'énergie est au contact des tissus situés dans ou autour du vagin et des organes génitaux externes du sujet, le dispositif étant en outre conçu pour utiliser les informations provenant du capteur afin de commander l'énergie transmise par l'élément de transmission d'énergie.
